# EUROPEAN PATENT SPECIFICATION

(11) **EP 3 166 644 B1**
(45) Date of publication and mention of the grant of the patent: **19.06.2019**
(21) Application number: 15738433.0
(22) Date of filing: 08.07.2015
(51) Int. Cl.: A61K 47/68, C07K 1/13

(54) **METHOD OF SYNTHESISING ADCS USING PHOTOCLEAVABLE LINKERS ON SOLID SUPPORT**
VERFAHREN ZUR SYNTHESE VON ADCS MITHILFE LICHTSPALTBARER LINKER AUF EINEM FESTEN TRÄGER
PROCÉDÉ DE SYNTHÈSE DE CONJUGUÉS ANTICORPS-MÉDICAMENT (ADCS) À L'AIDE DE LIANTS PHOTOCLIVABLES SUR UN SUPPORT SOLIDE

(30) Priority: 08.07.2014 GB 201412134
(43) Date of publication of application: 17.05.2017
(73) Proprietor: Adc Biotechnology Ltd, St. Asaph, Denbighshsire LL17 0JD (GB)
(72) Inventor: JOHNSON, Charles Ian Robert, St Asaph Denbighshire LL17 0JD (GB); JENKINS, Sara Louise, Ruthin Clwyd LL15 2RD (GB); MCKEE, Colin Martin, Colwyn Heights LL29 6DN (GB); EVANS, David John, Chester CH3 5RW (GB)
(74) Representative: HGF Limited
(86) International application number: PCT/GB2015/051974
(87) International publication number: WO 2016/005744

(56) References cited:
- WO-A1-2013/185078
- US-A1- 2002 168 644
- US-A1- 2007 212 731
- HAMMER R P ET AL: "PRACTICAL APPROACH TO SOLID-PHASE SYNTHESIS OF C-TERMINAL PEPTIDE AMIDES UNDER MILD CONDITIONS BASED ON A PHOTOLYSABLE ANCHORING LINKAGE1", INTERNATIONAL JOURNAL OF PEPTIDE AND PROTEIN RESEARCH, MUNKSGAARD, COPENHAGEN, DK, vol. 36, no. 1, 1 July 1990 (1990-07-01), pages 31-45, XP000134273, ISSN: 0367-8377

## Description

This invention relates to a solid phase method of synthesising biomolecule-drug-conjugates using photocleavable moieties. In particular, this invention relates to a solid phase method of synthesising antibody-drug-conjugates (ADCs) using photocleavable moieties. This invention also relates to intermediate methods of producing immobilised, chemically modified biomolecules, e.g. antibodies.

The invention also relates to intermediate products and compositions of the methods of the invention and biomolecule-drug-conjugates, in addition to other subject matter.

### BACKGROUND

Immunotoxins and antibody drug conjugates (ADCs) are proteinaceous drugs combining a target-specific binding domain with a drug molecule of sufficient potent toxicity that it may be classed as cytotoxic. Antibodies are the ideal biomolecule for this purpose creating a targeting system combining high specificity with high antigen affinity allowing the transportation of the cytotoxic drug direct to the site of desired administration. These drug constructs are potentially therapeutic against diseases, finding particular prevalence within oncology.

The main criteria of an Antibody Drug Conjugate (ADC) are that the toxin 'warhead' (drug) has activity at extremely low levels (picoM). Furthermore, it is advantageous to have efficacy towards tumour cells irrespective of the point in the cycle. For this purpose DNA active agents have found favour as toxin candidates as DNA damage, unless repairable, will drive apoptosis irrespective of the point in the cycle.

In principle, a suitable toxin for an ADC can be any moiety defined as a L01 ATC molecule ('Anatomical Therapeutic Chemical Classification System' where L01 is a subgroup defining antineoplastic and immunomodulating agents, defined by WHO Collaborating Centre for Drug Statistics Methodology). Alternatively, other moieties that may be categorised as suitable payloads for ADCs may be simply defined as anything that is toxic to cells once internalised. Most moieties falling in the latter category would lack sufficient potency to be effective. Hence, there is an industry trend to identify and exploit 'ultra-potency' materials.

An expert review on the rationale, design and effectiveness of immunotoxin and ADC research can be found within: J. Adair et al, Expert Opin. Biol. Ther., 2012, 12(9): P1191-206, G. Casi et al, Journal of Controlled Release, 2012, 161, 2, P422-428 and F. Dosio et al, Toxins, 2011, 3, P848-883.

A number of solution-phase methods can be used to manufacture biomolecule-drug-conjugates, e.g. antibody-drug-conjugates (ADCs). However, solution phase methods are themselves wasteful in terms of generating large volumes of waste and are problematic in terms of aggregation of the biomolecule-drug-conjugates during synthesis.

The first step in a solution-phase method for manufacturing biomolecule-drug-conjugates generally involves chemical modification or activation of the biomolecule. For example, where the biomolecule is an antibody, the antibody can be 'chemically modified' or 'activated' by reducing or partially reducing the antibody. A suitable process for partial reduction of antibodies is given in "Bioconjugate Techniques", page 96/97, Greg T. Hermanson, Academic Press; 2nd edition, 2008, ISBN-13: 978-0123705013. A reducing agent such as TCEP is generally employed in the reduction process.

After chemical modification or activation of the antibody, e.g. reduction, the next step is to remove any excess activation / chemical modification agent, e.g. excess reducing agent. This step is very time consuming as it is sometimes necessary to run the sample through a separation column multiple times. This can also be problematic in terms of degradation if stability of the biomolecule is an issue. The issue of purification of the chemically modified / activated biomolecule is particularly problematic if the process involves the full reduction of a ThiomAb with a large excess of a reducing agent.

After the above purification step, the chemically modified / activated, e.g. reduced, antibody is then conjugated with a drug moiety. The major problem with this step is the high probability of aggregation of the biomolecule-drug-conjugate. This is particularly problematic when highly hydrophobic drugs are employed in the process. Aggregation is a major problem as it can lead to unusable biomolecule-drug-conjugates. In the best case scenario, biomolecule-drug-conjugates contaminated with biomolecule-drug-conjugate aggregates must be further purified to remove the aggregates, which is both time consuming and very wasteful. A large proportion of the drug will be lost during purification as it forms part of the aggregated biomolecule-drug-conjugate. In the worst case the entire batch of biomolecule-drug-conjugate contaminated with biomolecule-drug-conjugate aggregate to such a high degree it is entirely unusable and must be disposed of.

Oncologists have been working on harnessing target-specific monoclonal antibodies to deliver cytotoxic drugs to the site of tumors as long as monoclonal antibodies have existed; nearly three decades. Up until now three classes of toxin have dominated the field. Namely, calicheamicins, maytansines and auristatins. These cytotoxic drug classes are all typically hydrophobic in nature. When conjugated to an antibody their presence increases the overall hydrophobicity of the antibody significantly and in some cases to the extent that hydrophobic interactions between conjugates leads to conjugate aggregation. The order of significance of this issue is Calicheamicin > Maytansine > Auristatin based on the knowledge that the processes for both Mylotarg and CMC-544 contain chromatographic aggregate removal steps. Approximately 50% of maytansine processes contain aggregate removal steps and very few auristatin processes contain aggregate removal steps.

More recently, toxins based on duocarmycins (www.syntarga.com) and pyrollebenzodiazepene (PBD) dimers (www.spirogen.com) have been conjugated to antibodies and are undergoing pre-clinical evaluation. These new classes of toxin are even more hydrophobic than their predecessor cytotoxin drug classes and are more prone to aggregation when conjugated to antibodies.

Significant efforts have been focussed on modulation of the hydrophobicity of the drug by incorporating hydrophilic linkers (Zhao et al, J. Med. Chem., 2011, 54, 10, 3606-3623). Where aggregate formation cannot be controlled developers have relied on well-known techniques for aggregate removal from protein based therapeutics. These include a range of different chromatographic separations including ion exchange, hydrophobic interaction, hydroxyapatite and others well known to those in the art. Undertaking such chromatographic purification techniques has the result of achieving adequate product quality but at the expense of process yield. When working with antibodies and antibody based therapeutics in the context of manufacturing physical loss of material through ambiguous, incidental side reactions or unwanted physiochemical interactions has a hugely significant financial impact.

Accordingly, the conventional solution-phase processes for manufacturing biomolecule-drug-conjugate are beset with difficulties and it would be desirable to provide an improved process for manufacturing biomolecule-drug-conjugates.

The present invention addresses one or more of the above issues with the conventional solution-phase methods.

### BRIEF SUMMARY OF THE DISCLOSURE

### Method of synthesising a biomolecule-drug-conjugate:

In accordance with the present invention there is provided a method of synthesising a biomolecule-drug-conjugate, the method comprising:
(i) either:
   a. contacting a biomolecule having a pendent -NRH group with a support that is functionalised with a photocleavable group under conditions suitable to covalently bond the biomolecule to the photocleavable group; or
   b. contacting a biomolecule having a pendent -NRH group with a photocleavable group under conditions suitable to covalently bond the biomolecule to the photocleavable group to produce a photocleavable group-biomolecule conjugate, and then immobilising the photocleavable group-biomolecule conjugate to a support;
   to provide an immobilised photocleavable group-biomolecule conjugate: 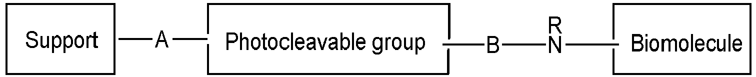
(ii) optionally contacting the immobilised photocleavable group-biomolecule conjugate with a chemical modification agent or activating agent to provide an immobilised photocleavable group-biomolecule conjugate that is chemically modified or activated at the biomolecule portion;
(iii) contacting the immobilised photocleavable group-biomolecule conjugate of step (i) or the chemically modified or activated immobilised photocleavable group-biomolecule conjugate of step (ii) with a drug component to form an immobilised photocleavable group-biomolecule-drug-conjugate: 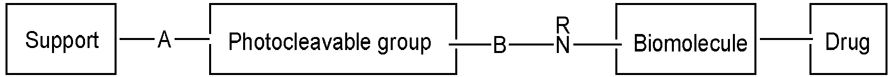
(iv) irradiating the immobilised photocleavable group-biomolecule-drug-conjugate of step (iii) with radiation thereby releasing the biomolecule-drug-conjugate 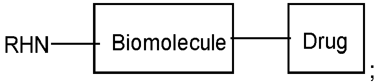 wherein:
   -A- is absent or is a spacer group;
   -B- is absent or is a spacer group;
   'Photocleavable group' comprises any residue that is able to cleave upon exposure to radiation to allow release of the biomolecule-drug-conjugate;
   R is selected from the group consisting of: H, C₁-C₈ alkyl, C₂-C₈ alkenyl, C₂-C₈ alkynyl, C₁-C₈ haloalkyl, -(CR^{a}R^{a})ₙ-C₃-C₆ cycloalkyl, -(CR^{a}R^{a})ₙ-C₃-C₆ heterocycloalkyl, -(CR^{a}R^{a})ₙ-C₃-C₆ halocycloalkyl, -(CR^{a}R^{a})ₙ-aryl, and -(CR^{a}R^{a})ₙ-heteroaryl;
   R^{a} is independently at each occurrence selected from: H, Me, CF₃ and F;
   n is an integer independently selected at each occurrence from 0, 1, 2 and 3; and
   'Drug' is a residue of a drug molecule selected from the group consisting of tubulin inhibitors and DNA interacting agents.

A key feature of the above method of the invention is that the mechanism by which the biomolecule is captured by the photocleavable group or the mechanism by which the biomolecule is released from the photocleavable group-biomolecule-drug-conjugate is not dependent on the identity of the biomolecule. In conventional covalent capture / release systems the biomolecule is captured by the capture moiety by a chemical reaction. The same can be said for the release of the biomolecule; the biomolecule is released from the capture moiety by a chemical reaction. The conditions that are utilised to 'drive' the chemical reaction are often unsuitable for certain biomolecules. For example, it is often necessary to utilise organic solvents or other reagents that are not compatible with biomolecules. The use of organic solvents with biomolecules such as antibodies can be particularly problematic. Therefore the applicability of conventional covalent capture / release systems can be limited to only a small number of biomolecules. In contrast, the present invention is broadly applicable to a plethora of biomolecules.

Additionally, the rate at which a chemical reaction proceeds can vary depending on the local environment at which the chemical reaction is taking place. The local environment can vary with the biomolecule. For example, the presence of hydrophobic or hydrophilic pockets in the biomolecule might affect the rate of a hydrolysis reaction or condensation reaction. In contrast, the present invention is not affected by the local environment and is therefore broadly applicable to biomolecules.

### Step (i):

In an embodiment, the step of providing an immobilised photocleavable group-biomolecule conjugate comprises contacting a biomolecule with a support that is functionalised with a photocleavable group under conditions suitable to covalently bond the biomolecule to the photocleavable group to produce the immobilised photocleavable group-biomolecule conjugate.

In an embodiment, the step of providing an immobilised photocleavable group-biomolecule conjugate comprises contacting a biomolecule with a photocleavable group under conditions suitable to covalently bond the biomolecule to the photocleavable group to produce a photocleavable group-biomolecule conjugate, and then immobilising the photocleavable group-biomolecule conjugate to a support to provide the immobilised photocleavable group-biomolecule conjugate.

In an embodiment, the step of contacting the biomolecule with a support that is functionalised with a photocleavable group comprises incubating the biomolecule with the support that is functionalised with a photocleavable group.

In an embodiment, the step of contacting the biomolecule with a photocleavable group comprises incubating the biomolecule with the photocleavable group.

In an embodiment, the step of contacting the biomolecule with a photocleavable group, whether itself supported or unsupported, comprises incubating the biomolecule with the photocleavable linker for a period of time of from about 20 minutes to about 3 days, optionally, from about 30 minutes to about 2 days and further optionally from about 1 hours to about 6 hours.

In an embodiment, the step of contacting the biomolecule with a photocleavable group, whether itself supported or unsupported, comprises incubating the biomolecule with the photocleavable linker at ambient temperature, e.g. at a temperature of from about 10°C to about 30°C, optionally about 15°C to about 25°C and further optionally about 20°C.

In an embodiment, the step of contacting the biomolecule with a photocleavable group, whether itself supported or unsupported, comprises adding the biomolecule in a suitable solvent, for example phosphate buffered saline (PBS), to the photocleavable linker. If the photocleavable linker is itself unsupported, the photocleavable linker may be dissolved in a suitable solvent. Examples of suitable solvents include DMF.

In embodiments in which the step of providing an immobilised photocleavable group-biomolecule conjugate comprises first contacting a biomolecule with photocleavable group to covalently bond the biomolecule to the photocleavable group and then subsequently immobilising the photocleavable group-biomolecule conjugate to a support, the immobilisation may be carried out by adding a solution of the photocleavable group-biomolecule conjugate to the support and incubating for a period of time. In an embodiment, the incubation lasts for a period of time of from about 10 minutes to about 1 day, optionally, from about 15 minutes to about 2 hours and further optionally from about 30 hours to about 1 hour. In an embodiment, the incubation occurs at ambient temperature, e.g. at a temperature of from about 10°C to about 30°C, optionally about 15°C to about 25°C and further optionally about 20°C.

In an embodiment, unreacted photocleavable linker is removed before moving onto the next step (step (ii)).

### Step (ii):

In one embodiment, step (ii) is omitted.

In an alternate embodiment, step (ii) is mandatory.

In an embodiment, the step of contacting the immobilised photocleavable group-biomolecule conjugate with a chemical modification agent or activating agent involves reducing the biomolecule. In an embodiment, the reduction of the biomolecule involves complete reduction. In an embodiment, the reduction of the biomolecule involves partial reduction. In an embodiment, the reduction of the biomolecule involves complete reduction followed by re-oxidation.

In an embodiment, the biomolecule is reduced by contacting it with a reducing agent such as tris(2-carboxyethyl)phosphine (TCEP), dithiothreitol (DTT), merceptoethylamine or other suitable reductant. Preferably the reducing agent is tris(2-carboxyethyl)phosphine (TCEP).

In an embodiment, the reduced biomolecule is re-oxidised by contacting it with an oxidising agent such as air, CuSO₄ or dehydroascorbic acid (DHAA). Preferably the oxidising agent is dehydroascorbic acid (DHAA).

In an embodiment, the process of reducing the biomolecule is carried out in a buffer solution such as phosphate buffered saline (PBS) or tris(hydroxymethyl)aminomethane (TRIS). In embodiments, the buffer may contain a chelating agent such as EDTA.

In an embodiment, the process of reducing the biomolecule is carried out at a pH of from about 5 to about 10, preferably from about 7 to about 9, preferably about 7.4 or about 8.5.

In an embodiment, the process of reducing the biomolecule is carried out in the presence of a chelating agent, such as EDTA.

In an embodiment, the process of reducing the biomolecule involves incubating the biomolecule with the reducing agent for a period of time of from about 20 minutes to about 3 days, optionally, from about 1 hour to about 2 days and further optionally from about 1 hour to about 12 hours, e.g. 1, 2, 3, 4, 5 or 6 hours.

In an embodiment, the process of reducing the biomolecule involves incubating the biomolecule with the reducing agent at ambient temperature, e.g. at a temperature of from about 10°C to about 30°C, optionally about 15°C to about 25°C and further optionally about 20°C.

In an embodiment, the step of contacting the immobilised photocleavable group-biomolecule conjugate with a chemical modification agent or activating agent involves reacting the biomolecule with a crosslinker moiety. For example, the crosslinker moiety could be an amine-to-sulfhydryl crosslinker, e.g. a crosslinker having an NHS-ester and a maleimide reactive group at opposite ends. This method of modifying or activating the biomolecule effectively results in a biomolecule-linker-drug-conjugate. Suitable cross-linkers are generally able to react with a primary amine group on the drug (via the reactive NHS ester end) and also react with a cysteine residue on the biomolecule (via the reactive maleimide end). In this particular example, the maleimide end will react with a cysteine in the immobilised biomolecule. An example of such a crosslinker is succinimidyl 4-(N-maleimidomethyl)cyclohexane-1-carboxylate (SMCC).

In an embodiment, the process of reacting with a crosslinker is carried out in a buffer solution such as phosphate buffered saline (PBS). Alternatively, the process of reacting with a crosslinker is carried out in a 'Modification Buffer' including a sodium phosphate buffer, NaCl and a chelating agent, such as EDTA.

In an embodiment, the process of reacting with a crosslinker is carried out at a pH of from about 7 to about 9, preferably from about 7 to about 8, preferably about 8.0.

In an embodiment, the process of reacting with a crosslinker is carried out in the presence of a chelating agent, such as EDTA.

In an embodiment, the process of reacting with a crosslinker involves incubating the biomolecule with the crosslinker for a period of time of from about 20 minutes to about 3 days, optionally, from about 1 hour to about 2 days and further optionally from about 6 hours to about 18 hours.

In an embodiment, the step of contacting the immobilised biomolecule with a chemical modification agent or an activating agent to provide a modified or activated, immobilised biomolecule involves reacting the biomolecule with Traut's reagent.

In an embodiment, the process of reacting with Traut's reagent is carried out in a buffer solution such as phosphate buffered saline (PBS).

In an embodiment, the process of reacting with Traut's reagent is carried out at a pH of from about 7 to about 9, preferably from about 7 to about 8, preferably about 8.0.

In an embodiment, the process of reacting with Traut's reagent is carried out in the presence of a chelating agent, such as EDTA.

In an embodiment, the process of reacting with Traut's reagent involves incubating the biomolecule with the reducing agent for a period of time of from about 20 minutes to about 3 days, optionally, from about 1 hour to about 2 days and further optionally from about 6 hours to about 18 hours.

In an embodiment, the activated, immobilised 'photocleavable group-biomolecule conjugate' is washed to remove any modification / activating agent. In an embodiment the washing involves rinsing with a buffer, optionally wherein the buffer is phosphate buffered saline (PBS). Other suitable buffers include: Potassium phosphate buffer; Sodium phosphate buffer; Sodium citrate buffer; Bis-Tris propane buffer; HEPES buffer; Sodium acetate buffer; Sodium citrate buffer; Cacodylic acid buffer; Ammonium acetate buffer; Imidazole buffer; Bicine buffer; and 2-(N-morpholino)ethanesulfonic acid (MES) buffer. For example, the immobilised biomolecule can be washed with a buffer solution such as phosphate buffered saline (PBS) at a pH of from about 7 to about 8, preferably about 7.4. Optionally, the rinsing of the activated, immobilised biomolecule is carried out in the presence of a chelating agent, such as EDTA. Another example of rinsing the activated, immobilised biomolecule involves rinsing the resin with a buffer such as PBS followed by a 'Conjugation Buffer' which includes sodium citrate, NaCl and a chelating agent such as EDTA.

### Step (iii):

In an embodiment, the step of contacting the immobilised photocleavable group-biomolecule conjugate of step (i) or the chemically modified or activated immobilised photocleavable group-biomolecule conjugate of step (ii) with a drug component to form an immobilised photocleavable group-biomolecule-drug-conjugate involves contacting with a drug component in a buffer solution as hereinbefore described with relation to step (ii).

In an embodiment, the step of contacting the immobilised photocleavable group-biomolecule conjugate of step (i) or the chemically modified or activated immobilised photocleavable group-biomolecule conjugate of step (ii) with a drug component to form an immobilised photocleavable group-biomolecule-drug-conjugate involves contacting with a drug component at a pH of from about 5 to about 8, preferably about 7 to about 8 and more preferably about 7.4.

In an embodiment, the step of contacting the immobilised photocleavable group-biomolecule conjugate of step (i) or the chemically modified or activated immobilised photocleavable group-biomolecule conjugate of step (ii) with a drug component to form an immobilised photocleavable group-biomolecule-drug-conjugate is carried out in the presence of a chelating agent, such as EDTA.

In an embodiment, the step of contacting the immobilised photocleavable group-biomolecule conjugate of step (i) or the chemically modified or activated immobilised photocleavable group-biomolecule conjugate of step (ii) with a drug component to form an immobilised photocleavable group-biomolecule-drug-conjugate involves incubating with drug component for a period of time of from about 10 minutes to about 3 days, optionally, from about 20 minutes to about 12 hours and further optionally from about 30 minutes to about 2 hours.

In an embodiment, the step of contacting the immobilised photocleavable group-biomolecule conjugate of step (i) or the chemically modified or activated immobilised photocleavable group-biomolecule conjugate of step (ii) with a drug component to form an immobilised photocleavable group-biomolecule-drug-conjugate involves incubating at ambient temperature, e.g. at a temperature of from about 10°C to about 30°C, optionally about 15°C to about 25°C and further optionally about 20°C.

In an embodiment, step of contacting the immobilised photocleavable group-biomolecule conjugate of step (i) or the chemically modified or activated immobilised photocleavable group-biomolecule conjugate of step (ii) with a drug component to form an immobilised photocleavable group-biomolecule-drug-conjugate involves incubating in a suitable solvent, for example DMA.

In an embodiment the reaction is quenched. Quenching of the reaction can be achieved by adding a suitable quenching agent such as N-acetyl cysteine. In an embodiment, the quenching lasts for a period of time of from about 10 minutes to about 1 day, optionally, from about 15 minutes to about 2 hours and further optionally from about 20 hours to about 1 hour. In an embodiment, the quenching occurs at ambient temperature, e.g. at a temperature of from about 10°C to about 30°C, optionally about 15°C to about 25°C and further optionally about 20°C.

In an embodiment, the immobilised photocleavable group-biomolecule-drug-conjugate is washed prior to the step of releasing the biomolecule-drug-conjugate (i.e. prior to step (iv)). The washing removes any unreacted drug component. In an embodiment the washing involves rinsing with a buffer, optionally wherein the buffer is phosphate buffered saline (PBS), and other solvent. Other suitable buffers include: Potassium phosphate buffer; Sodium phosphate buffer; Sodium citrate buffer; Bis-Tris propane buffer; HEPES buffer; Sodium acetate buffer; Sodium citrate buffer; Cacodylic acid buffer; Ammonium acetate buffer; Imidazole buffer; Bicine buffer; and 2-(N-morpholino)ethanesulfonic acid (MES) buffer. For example, the immobilised photocleavable group-biomolecule-drug-conjugate can be washed with a buffer solution such as phosphate buffered saline (PBS) and dimethylacetamide (DMA) at a pH of from about 5 to about 7. Optionally, the rinsing of the immobilised photocleavable group-biomolecule-drug-conjugate is carried out in the presence of a chelating agent, such as EDTA.

### Step (iv):

In an embodiment, step of irradiating the immobilised photocleavable group-biomolecule-drug-conjugate with radiation involves irradiating with UV light, for example light having a wavelength of from about 320 nm to about 400 nm. In an embodiment, the radiation has a wavelength of from about 350 nm to about 380 nm. In an embodiment, the radiation has a wavelength of from about 360 nm to about 370 nm, e.g. about 361, 362, 363, 364, 365, 366, 367, 368 or 369 nm. In an embodiment, the radiation has a wavelength of about 365 nm.

The radiation applied may comprise one or more wavelengths from the electromagnetic spectrum including x-rays (about 0.1 nm to about 10.0 nm), ultraviolet (UV) rays (about 10.0 nm to about 400 nm), visible light (about 400 nm to about 750 nm), infrared light (about 750 nm to about 0.1 cm), microwaves (about 0.1 cm to about 100 cm), and radio waves (about 100 cm to about 104 m). Multiple forms of radiation may also be applied simultaneously, in combination or coordinated in a step-wise fashion. Radiation exposure may be constant over a period of seconds, minutes or hours or varied with pulses at predetermined intervals. Radiation may be administered for less than about one hour, preferably less for than about 30 minutes, more preferably about 15 minutes. Alternatively, a period of time less than 15 minutes can be used, for example about ten minutes irradiation or a less than about one minute irradiation might also be sufficient to cleave the immobilised conjugate.

Typically, the radiation source is placed at a specified distance from the conjugate to be irradiated. For example, that distance may be empirically determined or calculated from the energy loss produced between the source and the target and the amount of energy emitted by the source.

### Support:

In an embodiment the support is a bead. The beads range in size from diameters of 10µm to 2000µm, preferably from 50µm to 1000µm, and most preferably from 75µm to 500µm. A preferred embodiment of the support is that the bead is porous in nature with a high surface area.

In an embodiment the support is a mobile support. Suitable mobile supports include:
- Polystyrene
- Polystyrene (PS-DVB) - Lightly cross-linked with divinylbenzene (0.1-5.0% DVB, termed Microporous)
- Polystyrene (PS-DVB) - Highly cross-linked with divinylbenzene (5-60% DVB, termed Macroporous)
- Polyethylene glycol
- Polyethylene glycol grafted polystyrene (PS-PEG co-polymer)
- Poly acrylamide
- Controlled Pore Glass (CPG) beads
- Silica
- Kieselguhr
- Polypropylene
- Poly(tetrafluoroethylene)
- Polyethylene
- Cellulose
- Poly methacrylate
- Functionalised Monoliths
- Functionalised Fibres
- Monolithic columns (such as Nikzad et al, OPRD, 2007, 11, 458-462)
- Agarose
- Sepharose
- Magnetic recoverable polymer beads

Of these, agarose, sepharose, cellulose, polyethylene glycol, silica, poly(styrene-oxyethylene) graft copolymers and polystyrene are particularly preferred. Agarose, Sepharose, poly(styrene-oxyethylene) graft copolymers and polystyrene are most preferred.

Suitable commercial mobile supports include:
- Hydroxymethyl PS (OH, microporous PS-1%DVB)
- Aminomethyl PS (NH2, microporous PS-1%DVB)
- NovaSyn TentaGel (NH2, grafted PEG-PS)
- Avidin Agarose (crosslinked 6% beaded agarose)
- NHS Agarose (N-hydroxysuccinimide, crosslinked 6% beaded agarose)
- NHS Sepharose (Highly cross-linked 4% agarose, spherical)

Suitable immobilised supports include:
- Membranes
- Stents
- Subcutaneous implants
- Cotton
- Multipin/Rod microtitre plates (such as Geysen et al, J. Immunological Methods, 1987, 102,259

Most preferred include Agarose based beaded supports. These beads are uniform spherical in nature and suitable for aqueous processes, which is particularly beneficial when working with antibodies. As these beads are uniform spherical in nature they are amenable to batch and flow technologies.

PS-DVB beads are based on a DVB-polystyrene matrix. When the % content of DVB in the bead composition is -2% it infers that the bead is highly swellable in organic solvents. As the bead is porous the internal matrix is fully accessible to reagents in this media. This type of bead composition can be termed 'Microporous'. However, in water based systems microporous beads do not swell. Consequently, the large majority of the functional sites that might bind to a biomolecule are not accessible and binding is reduced.

In an embodiment the bead is a macroporous bead. Many manipulations carried out on biomolecules are performed in water based systems. For PS-DVB the issue of water incompatibility in the context of the present invention is overcome by manipulating the bead composition. The % DVB content of the beads of the present invention is around 5-60%, for example 10-50%, 10-40%, 10-30%, or 20-25%. This results in the bead being more rigid and significantly less prone to swelling. Indeed, a bead of this composition does not swell at all in aqueous systems. This type of bead composition can be termed 'Macroporous'.

A 'macroporous' support contains a higher percentage composition of DVB compared to a microporus support. Although a 'macroporous' support has an identical internal porous matrix/network to a 'microporous' support its higher DVB composition fixes this matrix as a rigid network. Thus a 'macroporous' matrix does not undergo swelling. By removing the issue of adequate solvation/swelling a macroporous support may be used in any media environment; aqueous, semi-aqueous or organic. The internal matrix remains accessible regardless of the environmental media.

In a further embodiment the bead is a poly(styrene-oxyethylene) graft copolymer. Poly(styrene-oxyethylene) graft copolymer beads such as TentaGel are gelatinous resins constructed with a backbone of low crosslinked polystyrene grafted with polyoxyethylene (polyethylene glycol, PEG). This long PEG chain creates a spacer that effectively separates the terminal reactive site from the crosslinked backbone matrix. The PEG chain also infers a degree of hydrophilicity to the matrix. Poly(styrene-oxyethylene) graft copolymer beads are uniform spherical in nature and suitable for aqueous media permitting use in numerous biological applications. The beads may also be employed in organic and semi-organic media.

Similarly, a silica support can be used in aqueous, semi-aqueous or organic media as they are free from swelling. Thus, the derivatised support of the present invention also encompasses Silica beads. Silicas are highly suited to aqueous environments, readily available at tonne scale and relatively cheap.

The following beads can be derivatised with the photocleavable groups of described herein:
- Avidin Agarose (crosslinked 6% beaded agarose, Supplier: Pierce)
- Agarose NHS (N-hydroxysuccinimide, crosslinked 6% beaded agarose, Supplier: Pierce)
- Agarose (OH, NH2 , crosslinked 6% beaded agarose, Supplier: Pierce)
- Sepharose (OH, NH2, crosslinked 4% agarose, Supplier: Pierce)
- PuraBead (6% crosslinked agarose, Supplier: ProMetic BioSciences Ltd)
- PL-DVB (OH, microporous PS-1%DVB)
- Davisil LC1000Å (OH, irregular shaped silica, Supplier: Grace)
- MS-Gel D-50-1000Å (OH, spherical, porous, high purity silica)
- QuadraPure BZA (NH2, macroporous PS-20%DVB, Supplier: Johnson Matthey)
- Hydroxymethyl PS (OH, microporous PS-1%DVB, Supplier: Novabiochem)
- Aminomethyl PS (NH2, microporous PS-1%DVB, Supplier: Novabiochem)
- NovaSyn TentaGel (NH2, grafted PEG-PS, Supplier: Novabiochem).

The following support may also be derivatised in accordance with the present invention: QuadraPure™ (spherical, porous microporous & macroporous beads based DVB-PS) and QuadraSil™ (a porous, spherical silica) both sold by Johnson Matthey.

The derivatised support preferably comprises solid beads that are preferentially spherical in nature and of uniform size. Using a porous bead has the added advantage of increasing the surface area and therefore capacity (that is the amount of biomolecule that can be attached per gram of bead).

An important characteristic for purification of biomolecules is the pore size of any porous bead. Selectively may be inferred by the restriction of access of the biomolecule to the highly functional internal matrix. The beads of the present invention comprise pores that are of an amenable size for immobilising biomolecules as shown below.

| Pore Size | 100 Å | 300 Å | 1000 Å | 2000-4000 Å |
|---|---|---|---|---|
| Used for immobilising | Small organic molecules, small peptides, small nucleotides. | Polypeptides & globular proteins. | Fibrous proteins | Very large bio macromolecules |

The pore sizes of the derivatised support of the present invention is selected from about 100 Å, about 300 Å, about 1000 Å and about 2000-4000 Å. A pore size of 1000Å is appropriate for the purification of antibodies. For Agarose and Sepharose based spherical bead supports the extent of cross linking defines the porosity. Typically a 2-10% cross linking ratio is typical for commercial supports. Most preferred cross linking is 4-6%.

In an embodiment, the support is a capture resin.

### Biomolecule:

A biomolecule may be a chemical compound that naturally occurs in a living organism. A biological molecule may be a derivative of a chemical compound that naturally occurs in a living organism. A derivative of a biomolecule can be a biomolecule that has been altered chemically or genetically in a way which does not affects its biological activity. A derivative is a functional derivative. A derivative is a biologically effective analogue of the parent biomolecule.

A biomolecule can be a monomer or a polymer. A biomolecule can be a micromolecule or a macromolecule. A biomolecule might have a molecular weight of at least 50 K daltons, for example, 100, 200, 300, 400, 500, 600, 700, 800, 900 or 1000 K daltons.

In an embodiment the biomolecule is selected from the group comprising: antibodies, antibody fragments, modified antibodies, enzymes, proteins, peptides, polypeptides, modified peptides, peptide nucleic acids (PNAs), metalloproteins, peptide-oligonucleotide hybrids, amino acids, non-naturally occurring amino acids, diamino acids, synthetic amino acids, oligonucleotides, modified oligonucleotides, nucleotides, nucleosides, purines, pyrimidines, oligosaccharides, polysaccharide, disaccharides, monosaccharides, amino sugars, lipids, phospholipids, glycolipids, sterols, vitamins, hormones, steroids, neurotransmitters, carbohydrates, sugars, viruses, cells, active pharmaceutical ingredients (APIs), and precursor compounds or a derivatives of any of these. In an embodiment, the biomolecule is a vaccine, such as a virus for use in a vaccine.

In an embodiment the biomolecule is an antibody.

In an embodiment the biomolecule is an antibody fragments.

In an embodiment the biomolecule is a modified antibody.

In an embodiment the biomolecule is an enzyme.

In an embodiment the biomolecule is a protein.

In an embodiment the biomolecule is a peptide.

In an embodiment the biomolecule is a polypeptide.

In an embodiment the biomolecule is a modified peptide.

In an embodiment the biomolecule is a peptide nucleic acid (PNAs).

In an embodiment the biomolecule is a metalloprotein.

In an embodiment the biomolecule is a peptide-oligonucleotide hybrid.

In an embodiment the biomolecule is an amino acid.

In an embodiment the biomolecule is a non-naturally occurring amino acid.

In an embodiment the biomolecule is a diamino acid.

In an embodiment the biomolecule is a synthetic amino acid.

In an embodiment the biomolecule is an oligonucleotide.

In an embodiment the biomolecule is a modified oligonucleotide.

In an embodiment the biomolecule is a nucleotide.

In an embodiment the biomolecule is a nucleoside.

In an embodiment the biomolecule is a purine.

In an embodiment the biomolecule is a pyrimidine.

In an embodiment the biomolecule is an oligosaccharide.

In an embodiment the biomolecule is a polysaccharide

In an embodiment the biomolecule is a disaccharide.

In an embodiment the biomolecule is a monosaccharide.

In an embodiment the biomolecule is an amino sugar.

In an embodiment the biomolecule is a lipid.

In an embodiment the biomolecule is a phospholipid.

In an embodiment the biomolecule is a glycolipid.

In an embodiment the biomolecule is a sterol.

In an embodiment the biomolecule is a vitamin.

In an embodiment the biomolecule is a hormone.

In an embodiment the biomolecule is a steroid.

In an embodiment the biomolecule is a neurotransmitter.

In an embodiment the biomolecule is a carbohydrate.

In an embodiment the biomolecule is a sugar.

In an embodiment the biomolecule is a virus.

In an embodiment the biomolecule is a cell.

In an embodiment the biomolecule is an active pharmaceutical ingredient (API).

In an embodiment, the biomolecule is a vaccine.

In another embodiment, the biomolecule may be selected from any two or more of the above-mentioned biomolecules.

In a further embodiment, the biomolecule may bind selectively to photocleavable group. Thus, if two or more biomolecules are actually present in the original mixture (normally there being only a single biomolecule present in the original solution together with impurities) then only one biomolecule may be actually bound depending on conditions. Alternatively, more than one may be bound. The release of a particular biomolecule may be controlled selectively by changing the release conditions and/or release agent. The pH may be adjusted so that the derivatised support preferentially binds a first biomolecule rather than a second or further biomolecule. The isoelectric point (the pI) of a biomolecule may be exploited to determine a suitable pH for the binding of a particular biomolecule and the exclusion of one or more other biomolecules. Similarly, isoelectric point of a biomolecule may be exploited to determine a suitable pH for the release of a particular biomolecule whilst one or more different biomolecules remain bound to the derivatised support.

Preferably, in any of the aforementioned or later mentioned embodiments of the invention, the biomolecule is an antibody or an antibody fragment. Ideally, the biomolecule is any of the aforementioned or later mentioned embodiments of the invention is an antibody. The antibody may be an immunoglobulin (Ig). Five human immunoglobulin classes (IgG, IgA, IgM, IgD and IgE) exist. The term antibody encompasses monoclonal antibodies. The term antibody encompasses polyclonal antibodies. The term antibody encompasses antibody fragments so long as they exhibit the desired biological activity. The antibody can be a human antibody, an animal antibody, a murine antibody, a humanised antibody or a chimeric antibody that comprises human and animal sequences.

The basic unit of the antibody structure is a heterotetrameric glycoprotein complex of at least 20,000 daltons, for example about 150,000 daltons. An antibody might be at least 900 amino acids in length, for example 1400 amino acids in length. An antibody may composed of two identical light (L) chains and two identical heavy (H) chains, linked together by both noncovalent associations and by disulfide bonds. Each heavy and light chain also has regularly spaced intrachain disulfide bridges. Each heavy chain is of about 50,000 daltons. Each heavy chain is at least 300 amino acids in length, for example about 450 amino acids in length. The antibody may be a heavy chain only antibody. Each light chain is of about 20,000 daltons. Each light chain is at least 100 amino acids in length, for example about 250 amino acids in length.

An antibody biomolecule can contain two identical pairs of polypeptide chains, each pair having one light chain and one heavy chain. Each light chain and heavy chain in turn consists of two regions: a variable ("V") region involved in binding the target antigen, and a constant ("C") region that interacts with other components of the immune system. The light and heavy chain variable regions come together in 3 -dimensional space to form a variable region that binds the antigen (for example, a receptor on the surface of a cell).

In an embodiment the biomolecule is an antibody fragment. Antibody fragments comprise a portion of a full length antibody, generally the antigen binding or variable region thereof.

Examples of antibody fragments include Fab, pFc' F(ab')2, and scFv fragments; diabodies; linear antibodies; single-chain antibody biomolecules; and multispecific antibodies formed from antibody fragments. An antibody fragment might be at least 10 amino acids in length, for example an antibody fragment might be at least 20, 40, 60, 80, 100, 120, 140, 160, 180, 200, 220, 240, 260, 280 or 300 amino acids in length.

In an embodiment the biomolecule is an antibody

In an embodiment the biomolecule is a single monomeric variable antibody domain (Nanobody®).

In an embodiment the biomolecule is a modified antibody or a modified antibody fragment. By "modified antibody" or "modified antibody fragment" is meant an antibody that differs from a parent antibody by virtue of at least one amino acid modification. Or a modified antibody or modified antibody fragment refers to an antibody, which in comparison to the wild-type antibody, is different with respect to its size, or which is different with respect to its glycosylation but which has a similar affinity to its ligand as the wild-type antibody.

In an embodiment the biomolecule is an enzyme. An enzyme is a protein or a protein-based molecule that can catalyse a chemical reaction. An enzyme can also be an RNA based molecule, such as a ribozyme, that can catalyse a chemical reaction. An enzyme may be a globular protein. An enzyme may be at least 20, 30, 40, 50, 60, 80 or 100 amino acids in length. An enzyme may be between 50 and 3000 amino acids in length. An enzyme may be bound to a cofactor. An enzyme may be bound to an inhibitor.

In an embodiment the biomolecule is a peptide. As used herein, by "peptide" and "protein" mean at least two covalently attached amino acids linked by a peptidyl bond. A peptide comprises amino acid residues that are linked by covalent peptide (-C(O)NH-) or thiopeptide (-C(S)NH-) bonds. The term peptide includes proteins, polypeptides and oligopeptides. A peptide may be a globular protein or a fibrous protein. The peptidyl group may comprise naturally occurring amino acids and peptide bonds, or synthetic peptidomimetic structures, i.e. "analogs", such as peptoids. The amino acids may either be naturally occurring or non-naturally occurring or synthetic; as will be appreciated by those in the art. For example, homo-phenylalanine, citrulline, and norleucine are considered amino acids for the purposes of the invention, and both D- and L- (R or S) configured amino acids may be utilized. The term "peptide" encompasses purified natural products, or products which may be produced partially or wholly using recombinant or synthetic techniques.

The terms peptide and protein encompass biomolecules comprising amino acid chains of any length but preferably of at least 20 amino acids. A peptide may be at least 20, 30, 40, 50, 60, 80 or 100 amino acids in length. A peptide may be a full-length protein.

The terms peptide and protein may refer to an aggregate of a peptide such as a dimer or other multimer, a fusion peptide, a peptide variant, or derivative thereof. The term also includes modifications of the peptide, for example, peptides modified by glycosylation, acetylation, phosphorylation, pegylation, ubiquitination, and so forth. A peptide may comprise amino acids not encoded by a nucleic acid codon.

In an embodiment the biomolecule is a modified peptide. By "modification" is meant an amino acid substitution, insertion, and/or deletion in a polypeptide sequence or an alteration to a moiety chemically linked to a protein. For example, a modification may be an altered carbohydrate or PEG structure attached to a protein.

In an embodiment the biomolecule is an amino acid. By "amino acid" is intended to mean a moiety having an amine group and a carboxylic acid group. The amino acid residue may have one or more amine groups and one or more carboxylic acid groups. Thus the term "amino acid residue" is intended to include both natural and synthetic amino acids. The class of natural amino acids includes both proteinogenic amino acids and also naturally occurring non-proteinogenic amino acids. These naturally occurring non-proteinogenic amino acids are those that may be found, for example, in the body or in food stuffs, but which do not participate in protein biosynthesis. There are twenty-two proteinogenic amino acids and of the twenty-two, only twenty are directly encoded by the universal genetic code. The remaining two, selenocysteine and pyrrolysine, are incorporated into proteins by unique synthetic mechanisms. The invention is intended to encompass the twenty universally encoded amino acids plus the remaining two mentioned above. The term "amino acid residue" is therefore intended to include the following: Alanine, Cysteine, Aspartic Acid, Glutamic Acid, Phenylalanine, Glycine, Histidine, Isoleucine, Lysine, Leucine, Methionine, Asparagine, Proline, Glutamine, Arginine, Serine, Threonine, Valine, Tryptophan, Tyrosine, Selenocysteine and Pyrrolysine.

In addition to amino acid having a terminal carboxylic acid or amine group, the term is also intended to include: an amino acid alkyl ester (e.g. an amino acid C1-6 alkyl ester); an amino acid aryl ester; an N-alkylated amino acid (e.g. a C1-6 N-alkylated amino acid such as N-methylated amino acid or an N-methylcyclopropylated amino acid); an N,N-dialkylated amino acid (e.g. a C1-6 N,N-dialkylated amino acid, which can include N,N-dimethylcyclopropylated amino acids), preferably the N,N-dialkylated amino acid is an N,N-dimethylated amino acid; an N-acylated amino acid (e.g. a C1-6 N-acylated amino acid); an N-arylated amino acid; an N-alkylated amino acid ester; an N-acylated amino acid ester; an N-arylated amino acid ester; an O-alkylated amino acid (e.g. a C1-6 O-alkylated amino acid); an O-arylated amino acid; an O-acylated amino acid; an O-alkylated amino acid ester; an O-arylated amino acid ester; an O-acylated amino acid ester; an S-alkylated amino acid; an S-acylated amino acid; an S-arylated amino acid; an S-alkylated amino acid ester; an S-acylated amino acid ester; or an S-arylated amino acid ester. In other words, the invention also envisages amino acid derivatives such as those mentioned above which have been functionalized by simple synthetic transformations known in the art (e.g. as described in "Protective Groups in Organic Synthesis" by TW Greene and PGM Wuts, John Wiley & Sons Inc (1999), and references therein. Of course, in N,N-dialkylated amino acids, the alkyl groups may be the same or different.

In addition, the side chains of the above amino acids can be in either the (R) or the (S) configuration. In other words, both L- and D- amino acids are within the scope of the present invention, though the D- amino acids are of course not naturally occurring.

The term "amino acid" also includes non-proteinogenic amino acids such as amino acids which can be incorporated into proteins during translation (including pyrrolysine, ornithine and selenocysteine). The term "non-proteinogenic amino acid" also includes homologues of proteinogenic amino acids such as homoarginine. The term "non-proteinogenic amino acid" also includes beta amino acids such as beta Alanine. The term "amino acid" also includes lactam analogues of natural amino acids such as pyroglutamine.

In an embodiment the biomolecule is a non-naturally occurring amino acid. A non-naturally occurring amino acid is an organic compound which is an amino acid, but is not among those encoded by the standard genetic code, or incorporated into proteins during translation. Non-proteinogenic amino acids, thus, include amino acids or analogues of amino acids other than the 20 proteinogenic amino acids and include the D-isostereomers of proteinogenic amino acids. Examples of non-proteinogenic amino acids include: citrulline, homocitrulline, hydroxyproline, homoarginine, homoserine, homotyrosine, homoproline, ornithine, 4-amino-phenylalanine, sarcosine, biphenylalanine, homophenylalanine, 4-nitro-phenylalanine, 4-fluoro-phenylalanine, 2,3,4,5,6-pentafluoro-phenylalanine, norleucine, cyclohexylalanine, N-acetic acid, O-methyl serine, α-aminoisobutyric acid, N-methyl-alanine, N-methyl-glycine, N-methyl-glutamic acid, tert-butylglycine, α-aminobutyric acid, α-aminoisobutyric acid, acedic acid, 2-aminoisobutyric acid, 2-aminoindane-2-carboxylic acid, selenomethionine, lanthionine, dehydroalanine, γ-amino butyric acid, naphthylalanine, aminohexanoic acid, phenylglycine, pipecolic acid, 2,3-diaminoproprionic acid, tetrahydroisoquinoline-3-carboxylic acid, tert-leucine, tert-butylalanine, cyclohexylglycine, diethylglycine, dipropylglycine and derivatives thereof wherein the amine nitrogen has been mono- or di-alkylated. Other examples of non-proteinogenic amino acids include para amino benzoic acid (PABA), 5-amino salicylic acid (5-ASA) and 4-amino salicylic acid (4-ASA), Aib (aminobutyric acid), bAib (3-aminoisobutyric acid), Nva (norvaline), [beta]-Ala, Aad (2-aminoadipic acid), bAad (3-aminoadipic acid), Abu (2-aminobutyric acid), Gaba ([gamma]-aminobutyric acid), Acp (6-aminocaproic acid), Dbu (2,4-diaminobutyric acid), [alpha]-aminopimelic acid, TMSA (trimethylsilyl-Ala), alle(allo-isoleucine), Nle (norleucine), tert-Leu, Cit (citrulline), Orn, Dpm (2,2'-diaminopimelic acid), Dpr (2,3-diaminopropionic acid), [alpha]- or [beta]-Nal, Cha (cyclohexyl-Ala), hydroxyproline, Sar (sarcosine), etc.; cyclic amino acids; N-[alpha]-alkylated amino acids, e.g., MeGly (N-[alpha]-methylglycine), EtGly (N-[alpha]-ethylglycine), and EtAsn(N-[alpha]-ethylasparagine); and amino acids with two side chain substituents at the [alpha]-carbon, etc. Further examples of non-proteinogenic amino acids include dolaproine (Dap), dolaisoleuine (Dil), dolaphenine (Doe) and dolavaline (Dov); and unusual amino acids derived from natural sources.

In an embodiment the biomolecule is a synthetic amino acid. A synthetic amino acid is an amino acid that has been made synthetically. A synthetic amino acid is an amino acid that has not been synthesised within a living system. Examples of synthetic amino acids include nitro-phenylalanine and nitro-tyrosine.

In an embodiment the biomolecule is an oligonucleotide. The term "oligonucleotide" includes linear oligomers of nucleosides or analogs thereof, including deoxyribonucleosides, ribonucleosides. Usually oligonucleotides range in size from a few monomeric units, e.g. 2-4, to several hundreds of monomeric units. For example, an oligonucleotide may be at least 10, 50, 100, 150, 200, 400, 600, 800, 1000, or 2000 nucleotides in length. Oligonucleotides can be obtained from existing nucleic acid sources, including genomic or cDNA, or can be produced by synthetic methods. The nucleotide residues can be coupled to each other by any of the numerous known internucleoside linkages. Such internucleotide linkages include, without limitation, phosphodiester, phosphorothioate, phosphorodithioate, methylphosphonate, alkylphosphonate, alkylphosphonothioate, phosphotriester, phosphoramidate, siloxane, carbonate, carboalkoxy, acetamidate, carbamate, morpholino, borano, thioether, bridged phosphoramidate, bridged methylene phosphonate, bridged phosphorothioate, and sulfone internucleoside linkages.

In an embodiment the biomolecule is a modified oligonucleotide. The term "modified oligonucleotide" encompasses an oligonucleotide in which at least two of its nucleotides are covalently linked via a synthetic linkage, i.e., a linkage other than a phosphodiester linkage between the 5' end of one nucleotide and the 3' end of another nucleotide in which the 5' nucleotide phosphate has been replaced with any number of chemical groups. The term "modified oligonucleotide" also encompasses oligonucleotides having at least one nucleotide with a modified base and/or sugar, such as a 2'-O-substituted, a 5'- O-substituted and/or a 3'-O-substituted ribonucleotide. The term "modified oligonucleotide" also encompasses oligonucleotides having at least one substitution, insertion, and/or deletion of a base.

In an embodiment the biomolecule is a nucleotide. The term "nucleotide" generally refers to a nucleoside comprising a phosphorous- containing group attached to a sugar, usually ribose or deoxyribose.

In an embodiment the biomolecule is a nucleoside. A nucleoside generally refers to compounds consisting of a sugar, usually ribose or deoxyribose, and a purine or pyrimidine base.

In an embodiment the biomolecule is a purine. A purine is a nitrogenous base. A purine is a heterocyclic, aromatic organic compound consisting of a pyrimidine ring fused to an imidazole ring. Purines, include substituted purines and their tautomers.

In an embodiment the biomolecule is a pyrimidine. A pyrimidine is a single-ringed, crystalline organic base, C4H4N2, that forms uracil, cytosine, or thymine

In an embodiment the biomolecule is an oligosaccharide. Oligosaccharides are compounds comprising 2 to 10 monosaccharide residues.

In an embodiment the biomolecule is a polysaccharide. A polysaccharide is a polymer of monosaccharides containing 10 or more monosaccharide residues. For example a polysaccharide may comprise at least 10, 30, 50, 100, 500 or 1000 monosaccharide residues.

In an embodiment the biomolecule is a monosaccharide. A monosaccharide is a carbohydrate, such as a tetrose, pentose, or a hexose, that cannot be broken down to simpler sugars by hydrolysis. The term "monosaccharide" is intended to also cover derivatives of monosaccharides and oligosaccharides, in particular the reduced and oxidised forms thereof, such as sugar alcohols, e. g. sorbitol, mannitol, lactitol.

In an embodiment the biomolecule is an amino sugar. An amino sugar is a derivative of a sugar that contains an amine group in place of a hydroxyl group.

In an embodiment the biomolecule is a precursor compound. A precursor compound is a compound that participates in the chemical reaction that produces a biomolecule. A precursor compound may an intermediate compound in a chain of chemical reactions, from which a biomolecule is produced. A precursor compound may be a substance from which a more mature biomolecule is formed.

In an embodiment the biomolecule is a derivative of a biomolecule. The term derivative can mean a biological molecule that has been altered chemically or genetically in a way which does not affects its biological activity. A derivative is a functional derivative. A derivative is a biologically effective analogue of the parent biomolecule.

### Drug molecule:

The leading 'ultra-potency' (drug) candidates to date are defined in one of two categories: (i) tubulin inhibitors; and (ii) DNA interacting agents. Tubulin inhibitors modulate tubulin polymerization. DNA interacting agents target cellular DNA

In an embodiment the drug is a tubulin inhibitor.

In an embodiment, the tubulin inhibitor is selected from the group consisting of: (a) an auristatin; and (b) a maytansine derivative.

In an embodiment, the drug is an auristatin.

Auristatins include synthetic derivatives of the naturally occurring compound Dolastatin-10. Auristatins are a family of antineoplastic / cytostatic pseudopeptides. Dolastatins are structurally unique due to the incorporation of 4 unusual amino acids (Dolavaine, Dolaisoleuine, Dolaproine and Dolaphenine) identified in the natural biosynthetic product. In addition this class of natural product has numerous asymmetric centres defined by total synthesis studies by Pettit et al (US 4,978,744). It would appear from structure activity relationships that the Dolaisoleuine and Dolaproine residues appear necessary for antineoplastic activity (US 5,635,483 and US 5,780,588).

In an embodiment, the auristatin is selected from the group consisting of: Auristatin E (AE); Monomethylauristatin E (MMAE); Auristatin F (MMAF); vcMMAE; and vcMMAF.

In an embodiment, the drug is a maytansine or a structural analogue of maytansine.

In an embodiment, the drug is a maytansine.

Maytansines include structurally complex antimitotic polyketides. Maytansines are potent inhibitors of microtubulin assembly which leads towards apoptosis of tumour cells.

In an embodiment the maytansine is selected from the group consisting of: Mertansine (DM1); and a structural analogue of maytansine such as DM3 or DM4. Preferably, the drug is mertansine (DM1).

In an embodiment, the drug is DNA interacting agent. DNA interacting agents are known as 'ultra-potent' (drug) candidates.

In an embodiment, the DNA interacting agent is selected from the group consisting of: (a) calicheamicins, (b) duocarmycins and (c) pyrrolobenzodiazepines (PBDs).

In an embodiment, the drug is a calicheamicin.

Calicheamicin is a potent cytotoxic agent that causes double-strand DNA breaks, resulting in cell death. Calicheamicin is a naturally occurring enediyne antibiotic (A. L. Smith et al, J. Med. Chem., 1996, 39,11, 2103-2117). Calicheamicin was found in the soil microorganism *Micromonosporaechinospora.*

In an embodiment, the calicheamicin is calicheamicin gamma 1.

In an embodiment, the drug is a duocarmycin.

Duocarmycins are potent anti-tumour antibiotics that exert their biological effects through binding sequence-selectively in the minor groove of DNA duplex and alkylating the N3 of adenine (D. Boger, Pure & Appl. Chem., 1994, 66, 4, 837-844).

In an embodiment, the duocarmycin is selected from the group consisting of: Duocarmycin A; Duocarmycin B1; Duocarmycin B2; Duocarmycin C1; Duocarmycin C2; Duocarmycin D; Duocarmycin SA; Cyclopropylbenzoindole (CBI) duocarmycin; Centanamycin; Rachelmycin (CC-1065); Adozelesin; Bizelesin; and Carzelesin.

In an embodiment, the drug is a pyrrolobenzodiazepine.

Pyrrolobenzodiazepines (PBDs) are a class of naturally occurring anti-tumour antibiotics. Pyrrolobenzodiazepines are foundin *Streptomyces.* PBDs exert their anti-tumour activity by covalently binding to the DNA in the minor groove specifically at purine-guanine-purine units. They insert on to the N2 of guamine via an aminal linkage and, due to their shape, they cause minimal disruption to the DNA helix. It is believed that the formation of the DNA-PBD adduct inhibits nucleic acid synthesis and causes excision-dependent single and double stranded breaks in the DNA helix. As synthetic derivatives the joining of two PBD units together via a flexible polymethylene tether allows the PBD dimers to crosslink opposing DNA strands producing highly lethal lesions.

In an embodiment, the drug is a synthetic derivative of two pyrrolobenzodiazepines units joined together via a flexible polymethylene tether.

In an embodiment, the pyrrolobenzodiazepine is selected from the group consisting of: Anthramycin (and dimers thereof); Mazethramycin (and dimers thereof); Tomaymycin (and dimers thereof); Prothracarcin (and dimers thereof); Chicamycin (and dimers thereof); Neothramycin A (and dimers thereof); Neothramycin B (and dimers thereof); DC-81 (and dimers thereof); Sibiromycin (and dimers thereof); Porothramycin A (and dimers thereof); Porothramycin B (and dimers thereof); Sibanomycin (and dimers thereof); Abbeymycin (and dimers thereof); SG2000; and SG2285.

In an embodiment, the drug is a drug that targets DNA interstrand crosslinks through alkylation. A drug that targets DNA interstrand crosslinks through alkylation is selected from: a guanine-specific alkylating agent; and a adenine-specific alkylating agent.

In an embodiment, the drug is selected from the group consisting of: Netropsin; Distamycin; Lexitropsin; Tallimustine; Dibromotallimustine; PNU 157977; and MEN 10710.

In an embodiment, the drug is a Bis-(benzimidazole) carrier. Preferably, the drug is Hoechst 33258.

A guanine-specific alkylating agent is a highly regiospecific alkylating agents that reacts at specific nucleoside positions.

In an embodiment, the drug is a guanine-specific alkylating agent selected from the group consisting of: a G-N2 alkylators; and an A-N3 alkylator.

In an embodiment, the mitomycin is selected from: Mitomycin A; Mitomycin C; Porfiromycin; and KW-2149.

In an embodiment, the a carmethizole analogue is NSC 602668.

In an embodiment, the ecteinascidin analogue is Ecteinascidin 743.

Adenine-specific alkylating agents are regiospecific and sequence-specific minor groove alkylators reacting at the N3 of adenines in polypyrimidines sequences. Cyclopropaindolones and duocamycins may be defined as adenine-specific alkylators.

In an embodiment, the drug is a cyclopropaindolone analogue. Preferably, the drug is selceted from: adozelesin; and carzelesin.

In an embodiment, the drug is a benz[e]indolone. Preferably, the drug is selected from: CBI-TMI; and iso-CBI.

In an embodiment, the drug is bizelesin.

In an embodiment, the drug is a Marine Antitumor Drug. Marine Antitumor Drugs has been a developing field in the antitumor drug development arena (I. Bhatnagar et al, Mar. Drugs 2010, 8, P2702-2720 and T. L. Simmons et al, Mol. Cancer Ther. 2005, 4(2), P333-342). Marine organisms including sponges, sponge-microbe symbiotic association, gorgonian, actinomycetes, and soft coral have been widely explored for potential anticancer agents.

In an embodiment, the drug is selected from: Cytarabine, Ara-C; Trabectedin (ET-743); and EribulinMesylate.

In an embodiment, the EribulinMesylate is selected from: (E7389); Soblidotin (TZT 1027); Squalamine lactate; CemadotinPlinabulin (NPI-2358); Zalypsis; Tasidotin, Synthadotin; (ILX-651); Discodermolide; Hemiasterlin (E7974); LY355703; CRYPTO 52; Ecteinascidin 743; Squalamine; Cemadotin; Soblidotin; E7389; Discodermolide; HTI-286; LAF-389; Curacin A; Laulimalide; Vitilevuamide; Diazonamide; Eleutherobin; Sarcodictyin; Peloruside A; Variolins; and Halichondrin B.

The following drugs are also encompassed by the present invention: Tubulysins; Epothilone A, B, C, D, E, F.

Epothilones - constitute a class of non-taxane tubulin polymerisation agents and are obtained by natural fermentation of the myxobacterium *Sorangiumcellulosum.* These moieties possess potent cytotoxic activity which is linked to the stabilisation of microtubules and results in mitotic arrest at the G2/M transition. Epothilones have demonstrated potent cytotoxicity across a panel of cancer cell lines and has often exhibited greater potency than paclitaxel (X. :Pivot et al, European Oncology,2008;4(2), P42-45).

In an embodiment, the drug is tubulysin.

In an embodiment, the drug is epothilone.

The following drugs are also encompassed by the present invention. In an embodiment, the drug is selected from: Doxorubicin; Epirubicin; Esorubicin; Detorubicin; Morpholino-doxorubicin; Taxol; Melphalan; Vinblastine; Phomopsin A; Daunorubicin; Vinca alkaloids; Idarubicin; Cis-platin; Anthracyclines; Indolino-benzodiazepines; Actinomycin; Carminomycin; Podophyllotoxin; Etoposide; Hairpin polyamides; Etoposide phosphate; Vinblastine; Vincristine; Vindesine; TaxEsperamicin; and Ene-diynes.

In an embodiment, the drug is Doxorubicin.

In an embodiment, the drug is Epirubicin.

In an embodiment, the drug is Esorubicin.

In an embodiment, the drug is Detorubicin.

In an embodiment, the drug is Morpholino-doxorubicin.

In an embodiment, the drug is Taxol.

In an embodiment, the drug is Melphalan.

In an embodiment, the drug is Vinblastine.

In an embodiment, the drug is Phomopsin A.

In an embodiment, the drug is Daunorubicin.

In an embodiment, the drug is Vinca alkaloids.

In an embodiment, the drug is Idarubicin.

In an embodiment, the drug is Cis-platin.

In an embodiment, the drug is Anthracyclines.

In an embodiment, the drug is Indolino-benzodiazepines.

In an embodiment, the drug is Actinomycin.

In an embodiment, the drug is Carminomycin.

In an embodiment, the drug is Podophyllotoxin.

In an embodiment, the drug is Etoposide.

In an embodiment, the drug is Hairpin polyamide.

In an embodiment, the drug is Etoposide phosphate.

In an embodiment, the drug is Vinblastine.

In an embodiment, the drug is Vincristine.

In an embodiment, the drug is Vindesine.

In an embodiment, the drug is Esperamicin.

In an embodiment, the drug is Ene-diyne.

### Photocleavable groups:

There are a number of desirable characteristics that the photocleavable group should exhibit, which are discussed next. A photocleavable group should cleave from the remainder of the compound cleanly and uniformly and there should be a fast rate of release. Ideally, the reactive chromophore should cleave at a wavelength of 320 nm or higher to avoid any damage to biological components. Another important characteristic of the photocleavable group is that the photolysis products should not absorb light at the same wavelength as the wavelength at which the photocleavable group cleaves from the remainder of the compound.

### 2-nitrophenyl group:

In an embodiment, the photocleavable group is a 2-nitrophenzyl group of Formula IIa: 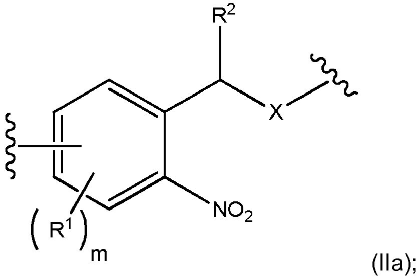 wherein:
each R¹ is a group independently selected from the group consisting of: substituted or unsubstituted C₁-C₄ alkyl, substituted or unsubstituted C₁-C₄ haloalkyl, substituted or unsubstituted C₁-C₄ alkoxyl and substituted or unsubstituted C₁-C₄ haloalkoxyl;
R² is selected from the group consisting of: H, substituted or unsubstituted C₁₋₄ alkyl and substituted or unsubstituted C₁-C₄ haloalkyl;
X is a group selected from the group consisting of: -O- and -S-; and
m is an integer of 0 to 3;
wherein each of the aforementioned alkyl, haloalkyl, alkoxyl and haloalkoxyl groups is optionally substituted, where chemically possible, by 1 to 5 substituents which are each independently at each occurrence selected from the group consisting of: oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, C₁-C₄-alkyl, C₂-C₄-alkenyl, C₂-C₄-alkynyl, C₁-C₄ haloalkyl, CR^{b}R^{b}NR^{a}R^{a}, and =CR^{b}CR^{b}R^{b}NR^{a}R^{a}; wherein R^{a} is independently at each occurrence selected from H, C₁-C₄ alkyl and C₁-C₄ haloalkyl; and R^{b} is independently at each occurrence selected from H, halogen, C₁-C₄ alkyl and C₁-C₄ haloalkyl.

In an embodiment, R¹ is selected from the group consisting of C₁-C₄ alkyl and C₁₋₄ alkoxy. Preferably, R¹ is C₁₋₄ alkoxy. Most preferably, R¹ is methoxy.

In an embodiment, m is 1.

In a preferred embodiment, the R¹ substituent is para to the -NO₂ substituent.

In an embodiment, R² is C₁₋₄ alkyl, e.g. C₁, C₂, C₃ or C₄ alkyl. Preferably R² is methyl.

In an embodiment, the immobilised photocleavable group-biomolecule conjugate has a structure:

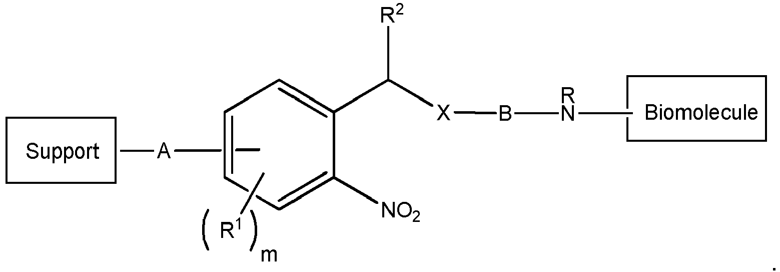

In an embodiment, the immobilised photocleavable group-biomolecule-drug conjugate has a structure:

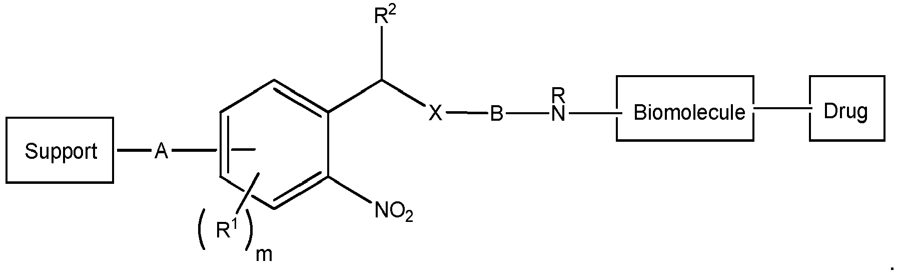

In an embodiment, X is -O-.

In an embodiment, B is selected from the group consisting of: -C(=O)- and -C(=S)-. In an embodiment, B is -C(=O)-.

In an embodiment, X is -O- and B is -C(=O)-. In this case, when the immobilised photocleavable group-biomolecule conjugate or the immobilised photocleavable group-biomolecule-drug conjugate is exposed to UV light, the biomolecule / biomolecule-drug conjugate are cleaved and the following by-products are produced:

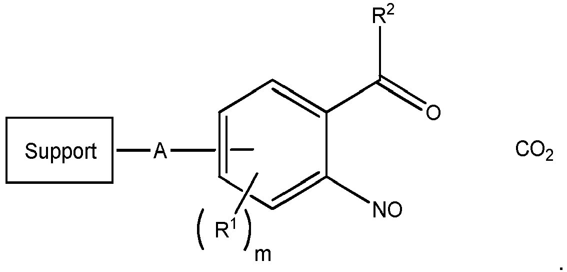

In an embodiment, -A- is present. In an embodiment, A comprises a linear chain of from 1 to 6 carbon atoms that may be interrupted by one or more heteroatoms selected from O, S or N.

In an embodiment -A- is: 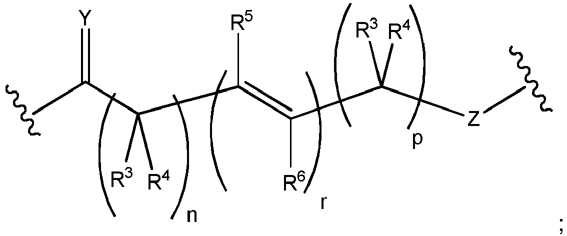
wherein the left hand side of -A- is bound to the support and the right hand side of -A- is bound to the photocleavable group;
R³ and R⁴ are each independently selected at each occurrence from the group comprising: hydrogen, halogen, hydroxy, C₁₋₆ alkoxy, C₁₋₆ alkyl C₁₋₆ alkoxy, -(CR⁷R⁸)_{q}OC(=O)R⁹, - (CR⁷R⁸)_{q}C(=O)R⁹, -C(=O)R⁹, C₁₋₆ alkyl, C₁₋₆ haloalkyl, aryl, -NR⁷R⁸ and -NR⁷(CO)R⁹; or together with the atom to which they are bonded, R³ and R⁴ may form a carbonyl, an ethylene or a C₃₋₆ cycloalkyl; and
R⁵ and R⁶ are each independently selected at each occurrence from the group comprising: hydrogen, halogen, hydroxy, C₁₋₆ alkoxy, C₁₋₆ alkyl C₁₋₆ alkoxy, -(CR⁷R⁸)_{q}OC(=O)R⁹,-(CR⁷R⁸)_{q}C(=O)R⁹, -C(=O)R⁹, C₁₋₆ alkyl, C₁₋₆ haloalkyl, aryl, -NR⁷R⁸ and -NR⁷(CO)R⁹;
R⁷ and R⁸ are each independently selected from the group comprising: H, C₁₋₆ alkyl, C₁₋₆ haloalkyl, C₃₋₈ cycloalkyl and phenyl;
R⁹ is selected from the group comprising: hydroxyl, C₁₋₆ alkyl, C₁₋₆ alkoxy, C₃₋₈ cycloalkyl and phenyl;
Y is selected from the group comprising: O and S;
Z is selected from the group comprising: -O-, -NH- and -S-;
n and p are each independently an integer of 0 - 16, provided that the sum of n and p is an integer of 0 - 16;
r is an integer of 0 - 2; and
q is an integer of 0 - 3;
wherein each of the aforementioned alkyl, haloalkyl, alkoxyl, aryl and cycloalkyl groups is optionally substituted, where chemically possible, by 1 to 5 substituents which are each independently at each occurrence selected from the group consisting of: oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, C₁-C₄-alkyl, C₂-C₄-alkenyl, C₂-C₄-alkynyl, C₁-C₄ haloalkyl, CR^{b}R^{b}NR^{a}R^{a}, and =CR^{b}CR^{b}R^{b}NR^{a}R^{a}; wherein R^{a} is independently at each occurrence selected from H, C₁-C₄ alkyl and C₁-C₄ haloalkyl; and R^{b} is independently at each occurrence selected from H, halogen, C₁-C₄ alkyl and C₁-C₄ haloalkyl.

In an embodiment, Y is O.

In an embodiment, R³ and R⁴ are each H.

In an embodiment, n is 3.

In an embodiment, r is 0.

In an embodiment, p is 0.

In an embodiment, Z is -O-.

### Benzoin group:

In an embodiment, the photocleavable group is a benzoin group of Formula IIb: 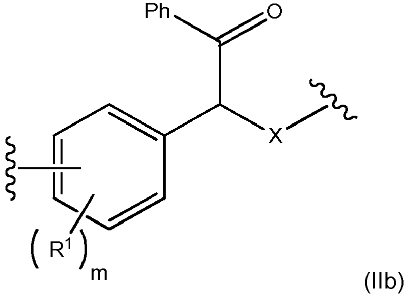 wherein:
each R¹ is a group independently selected from the group consisting of: OH, OR' and OCOR'; wherein R' is selected from the group consisting of: substituted or unsubstituted C₁₋₄ alkyl, substituted or unsubstituted C₁-C₄ haloalkyl, substituted or unsubstituted aryl and substituted or unsubstituted heteroaryl;
X is a group selected from the group consisting of: -O- and -S-; and
m is an integer of 0 to 4;
wherein each of the aforementioned alkyl, haloalkyl, aryl and heteroaryl groups is optionally substituted, where chemically possible, by 1 to 5 substituents which are each independently at each occurrence selected from the group consisting of: oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, C₁-C₄-alkyl, C₂-C₄-alkenyl, C₂-C₄-alkynyl, C₁-C₄ haloalkyl, CR^{b}R^{b}NR^{a}R^{a}, and =CR^{b}CR^{b}R^{b}NR^{a}R^{a}; wherein R^{a} is independently at each occurrence selected from H, C₁-C₄ alkyl and C₁-C₄ haloalkyl; and R^{b} is independently at each occurrence selected from H, halogen, C₁-C₄ alkyl and C₁-C₄ haloalkyl.

In an embodiment, R¹ is OR'. In an embodiment, R' is substituted or unsubstituted C₁₋₄ alkyl. In an embodiment, R' is methyl (i.e. R¹ is methoxy).

In an embodiment, m is 1.

In a preferred embodiment, the R¹ substituent is meta to the fixed substituent of the benzoin group of Formula IIb.

In a preferred embodiment, the photocleavable group has a structure:

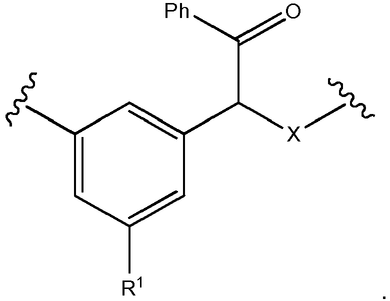

In an embodiment, the immobilised photocleavable group-biomolecule conjugate has a structure:

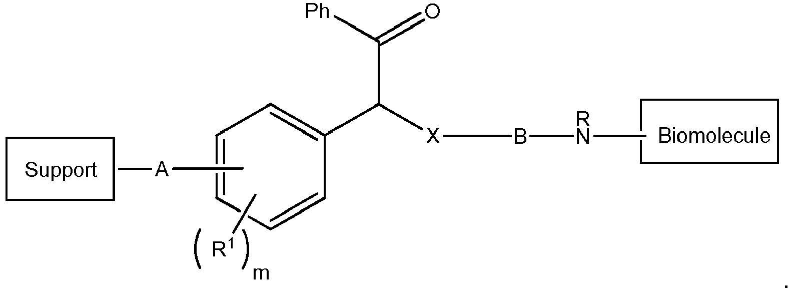

In an embodiment, the immobilised photocleavable group-biomolecule-drug conjugate has a structure:

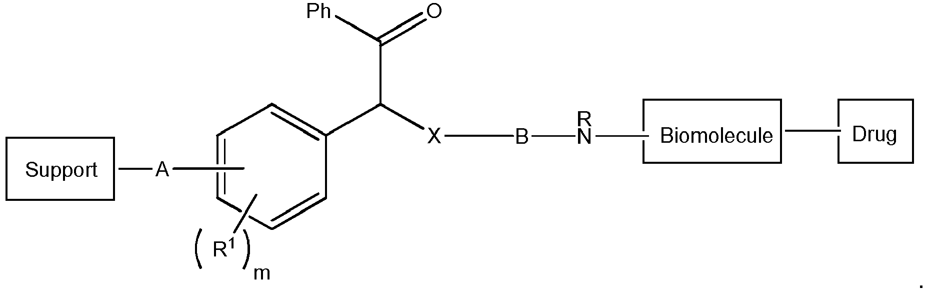

In an embodiment, X is -O-.

In an embodiment, B is selected from the group consisting of: -C(=O)- and -C(=S)-. In an embodiment, B is -C(=O)-.

In an embodiment, X is -O- and B is -C(=O)-. In this case, when the immobilised photocleavable group-biomolecule conjugate or the immobilised photocleavable group-biomolecule-drug conjugate is exposed to UV light, the biomolecule / biomolecule-drug conjugate are cleaved and the following by-products are produced:

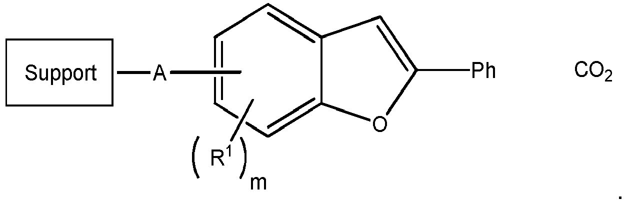

In an embodiment, -A- is absent.

### Phenacyl groups:

In an embodiment, the photocleavable group is a phenacyl group of Formula IIc: 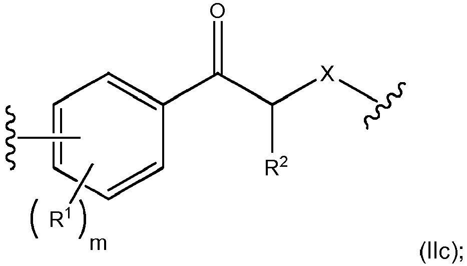 wherein:
each R¹ is a group independently selected from the group consisting of: OH, OR' and OCOR'; wherein R' is selected from the group consisting of: substituted or unsubstituted C₁₋₄ alkyl, substituted or unsubstituted C₁-C₄ haloalkyl, substituted or unsubstituted aryl and substituted or unsubstituted heteroaryl;
R² is selected from the group consisting of: H, C₁₋₄ alkyl and C₁-C₄ haloalkyl;
X is a group selected from the group consisting of: -O- and -S-; and
m is an integer of 0 to 4;
wherein each of the aforementioned alkyl, haloalkyl, aryl and heteroaryl groups is optionally substituted, where chemically possible, by 1 to 5 substituents which are each independently at each occurrence selected from the group consisting of: oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, C₁-C₄-alkyl, C₂-C₄-alkenyl, C₂-C₄-alkynyl, C₁-C₄ haloalkyl, CR^{b}R^{b}NR^{a}R^{a}, and =CR^{b}CR^{b}R^{b}NR^{a}R^{a}; wherein R^{a} is independently at each occurrence selected from H, C₁-C₄ alkyl and C₁-C₄ haloalkyl; and R^{b} is independently at each occurrence selected from H, halogen, C₁-C₄ alkyl and C₁-C₄ haloalkyl.

In an embodiment, R¹ is OR'. In an embodiment, R' is substituted or unsubstituted C₁₋₄ alkyl. In an embodiment, R' is methyl (i.e. R¹ is methoxy).

In an embodiment, m is 1.

In a preferred embodiment, the R¹ substituent is para to the fixed substituent of the benzoin group of Formula IIc. In a preferred embodiment, the R¹ substituent is ortho to the fixed substituent of the benzoin group of Formula IIc.

In a preferred embodiment, the photocleavable group has a structure:

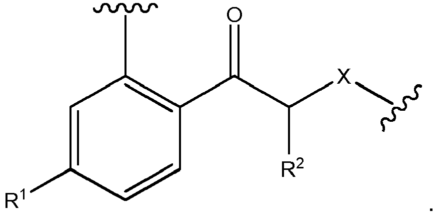

In an embodiment, R² is H. In an embodiment, R² is C₁₋₄ alkyl, preferably methyl.

In an embodiment, the immobilised photocleavable group-biomolecule conjugate has a structure:

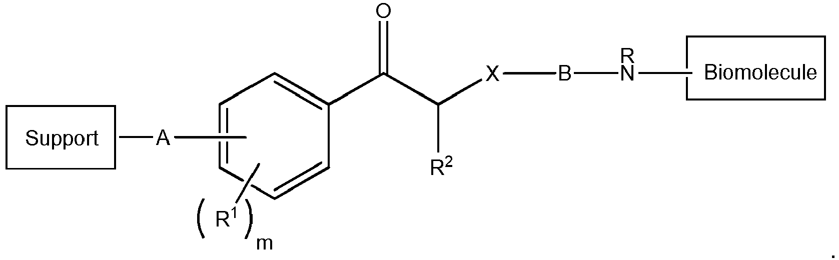

In an embodiment, the immobilised photocleavable group-biomolecule-drug conjugate has a structure:

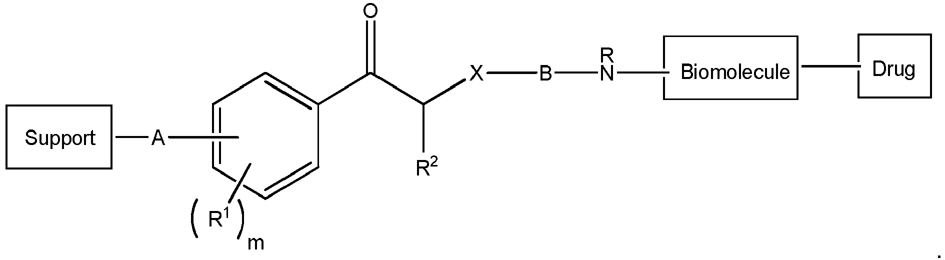

In an embodiment, X is -O-.

In an embodiment, B is selected from the group consisting of: -C(=O)- and -C(=S)-. In an embodiment, B is -C(=O)-.

In an embodiment, X is -O- and B is -C(=O)-. In this case, when the immobilised photocleavable group-biomolecule conjugate or the immobilised photocleavable group-biomolecule-drug conjugate is exposed to UV light, the biomolecule / biomolecule-drug conjugate are cleaved and the following by-products are produced:

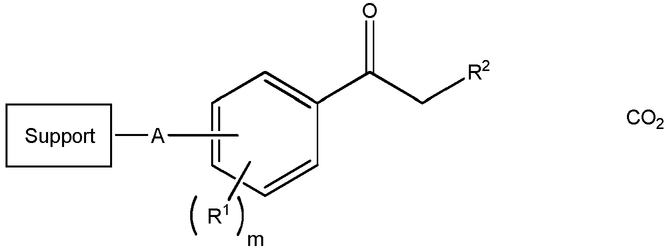

In an embodiment, -A- is absent.

### 4-hydroxy phenacyl groups:

In an embodiment, the photocleavable group is a 4-hydroxy phenacyl group of Formula IId: 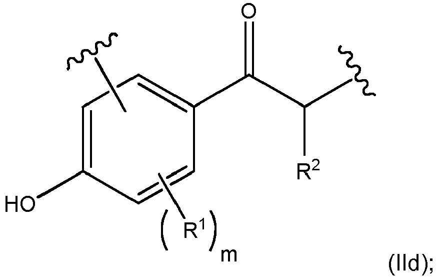 wherein:
each R¹ is a group independently selected from the group consisting of: OH, OR' and OCOR'; wherein R' is selected from the group consisting of: substituted or unsubstituted C₁₋₄ alkyl, substituted or unsubstituted C₁-C₄ haloalkyl, substituted or unsubstituted aryl and substituted or unsubstituted heteroaryl;
R² is selected from the group consisting of: C₁₋₄ alkyl and C₁-C₄ haloalkyl; and
m is an integer of 0 to 3;
wherein each of the aforementioned alkyl, haloalkyl, aryl and heteroaryl groups is optionally substituted, where chemically possible, by 1 to 5 substituents which are each independently at each occurrence selected from the group consisting of: oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, C₁-C₄-alkyl, C₂-C₄-alkenyl, C₂-C₄-alkynyl, C₁-C₄ haloalkyl, CR^{b}R^{b}NR^{a}R^{a}, and =CR^{b}CR^{b}R^{b}NR^{a}R^{a}; wherein R^{a} is independently at each occurrence selected from H, C₁-C₄ alkyl and C₁-C₄ haloalkyl; and R^{b} is independently at each occurrence selected from H, halogen, C₁-C₄ alkyl and C₁-C₄ haloalkyl.

In an embodiment, m is 0.

In an embodiment, R² is H. In an embodiment, R² is C₁₋₄ alkyl, preferably methyl.

In an embodiment, the immobilised photocleavable group-biomolecule conjugate has a structure:

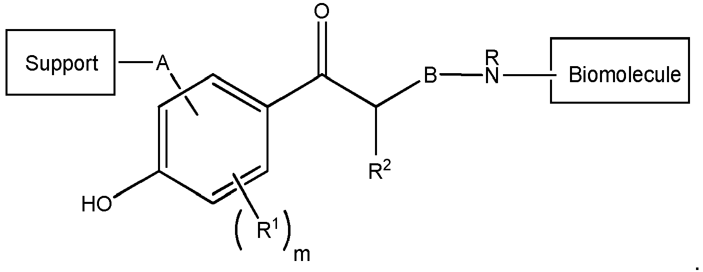

In an embodiment, the immobilised photocleavable group-biomolecule-drug conjugate has a structure:

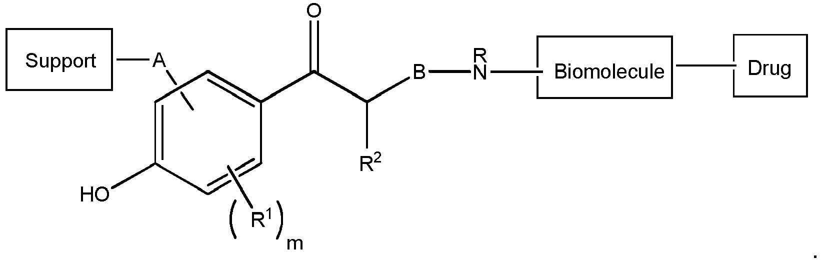

In an embodiment, B is absent. In this case, when the immobilised photocleavable group-biomolecule conjugate or the immobilised photocleavable group-biomolecule-drug conjugate is exposed to UV light, the biomolecule / biomolecule-drug conjugate are cleaved and the following by-product is produced: 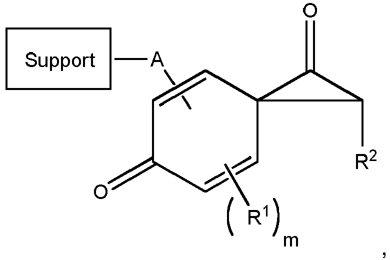 which may then yield:

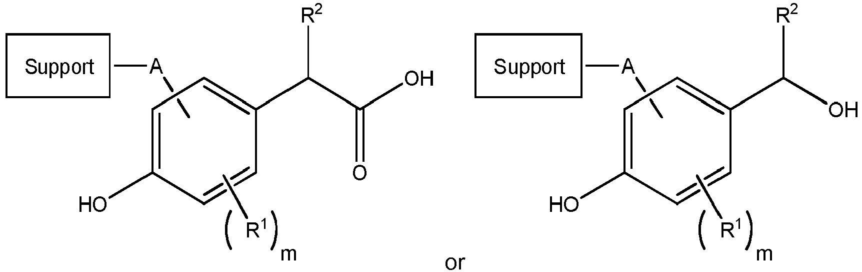

In an embodiment, -A- is absent.

### (2-hydroxyphenyl)acrylic acid group:

In an embodiment, the photocleavable group is a (2-hydroxyphenyl)acrylic acid group of Formula IIe: 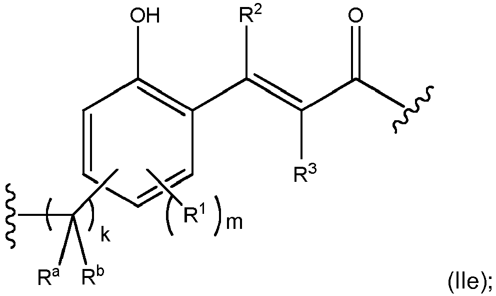 wherein:
each R¹ is a group independently selected from the group consisting of: OH, OR' and OCOR'; wherein R' is selected from the group consisting of: substituted or unsubstituted C₁₋₄ alkyl, substituted or unsubstituted C₁-C₄ haloalkyl, substituted or unsubstituted aryl and substituted or unsubstituted heteroaryl;
R² and R³ are each independently selected from the group consisting of: H, C₁₋₄ alkyl and C1-C4 haloalkyl;
R^{a} and R^{b} are each independently selected from the group consisting of: H, C₁₋₄ alkyl and C1-C4 haloalkyl;
k is an integer of 0 to 3; and
m is an integer of 0 to 3;
wherein each of the aforementioned alkyl, haloalkyl, aryl and heteroaryl groups is optionally substituted, where chemically possible, by 1 to 5 substituents which are each independently at each occurrence selected from the group consisting of: oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, C₁-C₄-alkyl, C₂-C₄-alkenyl, C₂-C₄-alkynyl, C₁-C₄ haloalkyl, CR^{b}R^{b}NR^{a}R^{a}, and =CR^{b}CR^{b}R^{b}NR^{a}R^{a}; wherein R^{a} is independently at each occurrence selected from H, C₁-C₄ alkyl and C₁-C₄ haloalkyl; and R^{b} is independently at each occurrence selected from H, halogen, C₁-C₄ alkyl and C₁-C₄ haloalkyl.

In an embodiment, m is 0.

In an embodiment, k is 0 or 1.

In an embodiment, R² is H.

In an embodiment, R³ is H.

In an embodiment, R^{a} is H.

In an embodiment, R^{b} is H.

In an embodiment, the immobilised photocleavable group-biomolecule conjugate has a structure:

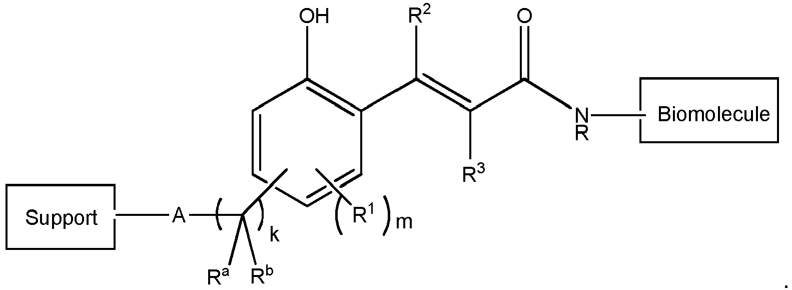

In an embodiment, the immobilised photocleavable group-biomolecule-drug conjugate has a structure:

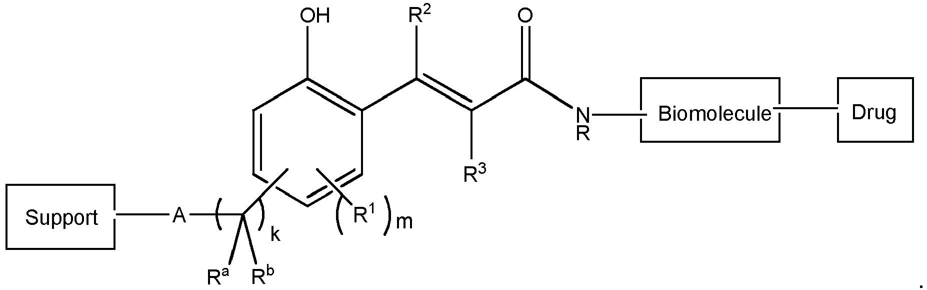

When the immobilised photocleavable group-biomolecule conjugate or the immobilised photocleavable group-biomolecule-drug conjugate is exposed to UV light, the biomolecule / biomolecule-drug conjugate are cleaved and the following by-products are produced:

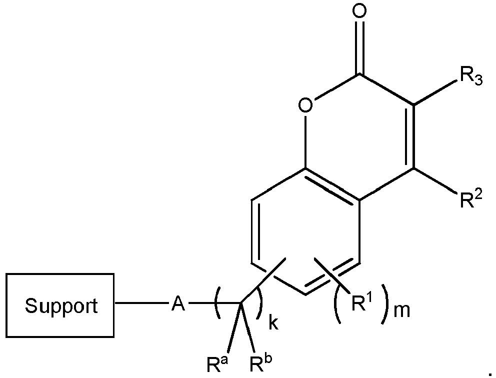

In an embodiment, -A- is absent.

In an embodiment, -A- is present. In an embodiment, A is comprises a linear chain of from 1 to 6 carbon atoms that may be interrupted by one or more heteroatoms selected from O, S or N.

In an embodiment -A- is: 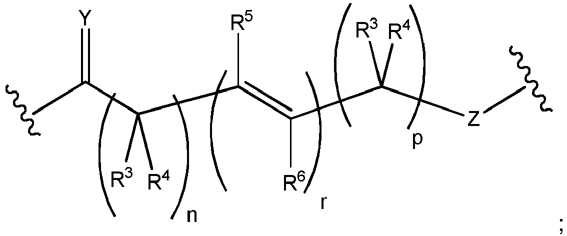
wherein the left hand side of -A- is bound to the support and the right hand side of -A- is bound to the photocleavable group;
R³ and R⁴ are each independently selected at each occurrence from the group comprising: hydrogen, halogen, hydroxy, C₁₋₆ alkoxy, C₁₋₆ alkyl C₁₋₆ alkoxy, -(CR⁷R⁸)_{q}OC(=O)R⁹,-(CR⁷R⁸)_{q}C(=O)R⁹, -C(=O)R⁹, C₁₋₆ alkyl, C₁₋₆ haloalkyl, aryl, -NR⁷R⁸ and -NR⁷(CO)R⁹; or together with the atom to which they are bonded, R³ and R⁴ may form a carbonyl, an ethylene or a C₃₋₆ cycloalkyl; and
R⁵ and R⁶ are each independently selected at each occurrence from the group comprising: hydrogen, halogen, hydroxy, C₁₋₆ alkoxy, C₁₋₆ alkyl C₁₋₆ alkoxy, -(CR⁷R⁸)_{q}OC(=O)R⁹,-(CR⁷R⁸)_{q}C(=O)R⁹, -C(=O)R⁹, C₁₋₆ alkyl, C₁₋₆ haloalkyl, aryl, -NR⁷R⁸ and -NR⁷(CO)R⁹;
R⁷ and R⁸ are each independently selected from the group comprising: H, C₁₋₆ alkyl, C₁₋₆ haloalkyl, C₃₋₈ cycloalkyl and phenyl;
R⁹ is selected from the group comprising: hydroxyl, C₁₋₆ alkyl, C₁₋₆ alkoxy, C₃₋₈ cycloalkyl and phenyl;
Y is selected from the group comprising: O and S;
Z is selected from the group comprising: -O-, -NH- and -S-;
n and p are each independently an integer of 0 - 16, provided that the sum of n and p is an integer of 0 - 16;
r is an integer of 0 - 2; and
q is an integer of 0 - 3;
wherein each of the aforementioned alkyl, haloalkyl, alkoxyl, aryl and cycloalkyl groups is optionally substituted, where chemically possible, by 1 to 5 substituents which are each independently at each occurrence selected from the group consisting of: oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, C₁-C₄-alkyl, C₂-C₄-alkenyl, C₂-C₄-alkynyl, C₁-C₄ haloalkyl, CR^{b}R^{b}NR^{a}R^{a}, and =CR^{b}CR^{b}R^{b}NR^{a}R^{a}; wherein R^{a} is independently at each occurrence selected from H, C₁-C₄ alkyl and C₁-C₄ haloalkyl; and R^{b} is independently at each occurrence selected from H, halogen, C₁-C₄ alkyl and C₁-C₄ haloalkyl.

In an embodiment, Y is O.

In an embodiment, R³ and R⁴ are each H.

In an embodiment, n is 0.

In an embodiment, r is 0.

In an embodiment, p is 0.

In an embodiment, Z is -NH-.

The present invention also includes the synthesis of all pharmaceutically acceptable isotopically-labelled compounds of the above formulae wherein one or more atoms are replaced by atoms having the same atomic number, but an atomic mass or mass number different from the atomic mass or mass number most commonly found in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds, for example, those incorporating a radioactive isotope, are useful in drug and/or substrate tissue distribution studies. The radioactive isotopes tritium, i.e. ³H, and carbon-14, i.e. ¹⁴C, are particularly useful for this purpose in view of their ease of incorporation and ready means of detection.

Substitution with heavier isotopes such as deuterium, i.e. ²H, may afford certain therapeutic advantages resulting from greater metabolic stability, for example, increased in vivo half-life or reduced dosage requirements, and hence may be preferred in some circumstances. Deuterated compounds are particularly useful in the compounds of the invention.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labelled compounds can generally be prepared by conventional techniques known to those skilled in the art or by processes analogous to those described using an appropriate isotopically-labelled reagent in place of the non-labelled reagent previously employed.

### EXAMPLES:

### Example 1. Reaction of BSA and Linker 1

The following approaches can be used for reacting BSA or Herceptin with commercially available photocleavable biotin (PC-Biotin, Ambergen Inc. - hereafter defined as 'Linker 1') then subsequent cleavage using photolysis conditions to yield the protein or antibody.

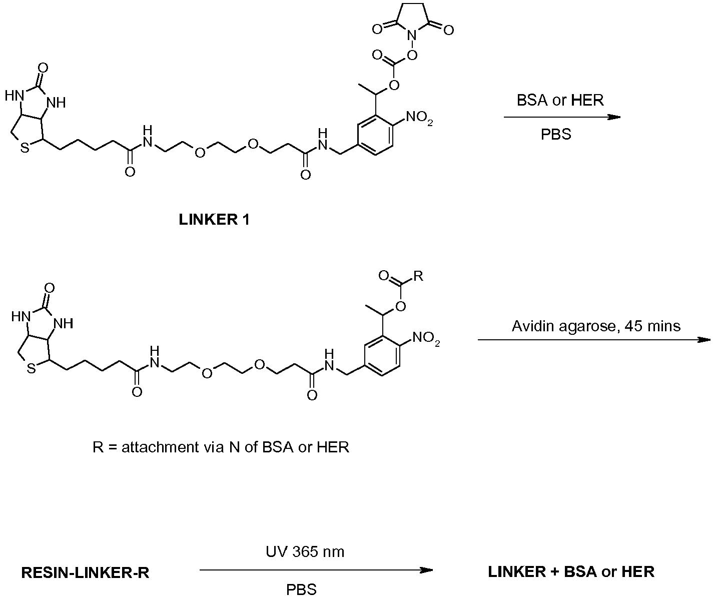

Prepare a 50mmole stock solution of Linker 1 in anhydrous DMF. Prepare a 1mg/ml solution of BSA in PBS (pH7.4). An aliquot of the BSA solution (0.5ml) was incubated with 10-20 molar equivalents excess of Linker 1 stock solution. The resultant mixture was then incubated for 60 minutes at ambient temperature with gentle mixing. Unreacted linker was removed by passing through an Illustra Nap5 (GE Healthcare) disposable buffer exchange column, using PBS (pH7.4) as eluent. The filtrate containing Linker-BSA was collected for subsequent immobilisation on to avidin agarose resin.

### Example 2. Immobilisation of Linker-BSA complex on to avidin agarose resin

Use approx. 3mg of protein to 1ml (2ml slurry) of wetted avidin agarose resin (6% crosslinked beaded agarose). The solution of purified Linker-BSA from Example 1 was charged to 166µls of settled avidin agarose resin (50% slurry in 0.02% sodium azide solution) which had been washed thoroughly with 5 aliquots of PBS (pH7.4). The resultant suspension was incubated for 45 minutes at ambient temperature with gentle mixing. After this duration the resin-Linker-BSA complex was filtered and washed sequentially with 10 aliquots of fresh PBS (pH7.4) to remove any superfluous reagents.

Verification of immobilisation of Linker-BSA to avidin resin was confirmed by SEC HPLC and UV (λ 280nm) of the resin supernatant, which showed no evidence of BSA after treatment with the resin.

### Example 3. Photolytic cleavage of BSA from avidin agarose resin

Cleavage of BSA from the resin-Linker-BSA complex was achieved by exposure to UV light (long wave 365nm). Washed resin-Linker-BSA complex was transferred to a glass vial and re-suspended in PBS (pH7.4). The suspension was then subjected to exposure to UV light for up to 15 minutes. The supernatant collected by filtration and analysed by chromatographic means.

Verification of cleavage was confirmed by SEC HPLC and UV which by comparison with a known standard of BSA clearly demonstrated cleavage and recovery of BSA from the resin. Cleavage yield 45% calculated from UV at 280nm.

### Example 4. Reaction of Herceptin and Linker 1

Prepare a 50mmole stock solution of Linker 1 in anhydrous DMF. Prepare a 1mg/ml solution of Herceptin antibody in PBS (pH7.4). An aliquot of the Herceptin solution (0.5ml) was incubated with 10-20 molar equivalents excess of Linker 1 stock solution. The resultant mixture was then incubated for 60 minutes at ambient temperature with gentle mixing. Unreacted linker was removed by passing through an Illustra Nap5 disposable buffer exchange column, using PBS (pH7.4) as eluent. The filtrate containing Linker-Herceptin was collected for subsequent immobilisation on to avidin agarose resin.

### Example 5. Immobilisation of Linker-Herceptin complex onto avidin agarose resin

Use approx. 3mg of antibody to 1ml (2ml slurry) of wetted avidin agarose resin (6% cross linked beaded agarose). The solution of purified Linker-Herceptin from Example 4 was charged to 166µls of settled avidin agarose resin (50% slurry in 0.02% sodium azide solution) which had been washed thoroughly with 5 aliquots of PBS (pH7.4). The resultant suspension was incubated for 45 minutes at ambient temperature with gentle mixing. After this duration the resin-Linker-Herceptin complex was filtered and washed sequentially with 10 aliquots of fresh PBS (pH7.4) to remove any superfluous reagents.

Verification of immobilisation of Linker-Herceptin to avidin resin was confirmed by SEC HPLC and UV (λ280nm) of the resin supernatant, which showed no evidence of Herceptin after treatment with the resin.

### Example 6. Photolytic cleavage of Herceptin from avidin agarose resin

Cleavage of Herceptin from the resin-Linker-Herceptin complex was achieved by exposure to UV light (long wave 365nm). Washed resin-Linker-Herceptin complex was transferred to a glass vial and re-suspended in PBS (pH7.4). The suspension was then subjected to exposure to UV light for up to 15 minutes. The supernatant collected by filtration and analysed by chromatographic means.

Verification of cleavage was confirmed by SEC HPLC and UV which by comparison with a known standard of Herceptin clearly demonstrated cleavage and recovery of Herceptin from the resin. Cleavage yield 47% calculated from UV at 280 nm.

### Example 7. Solid phase synthesis of an activated antibody-linker conjugate

The following procedure describes the synthesis and assembly of an Antibody Drug Conjugate (ADC) upon an avidin agarose solid phase platform wherein release of the assembled ADC is performed selectively by the operator via photolysis, back in to solution.

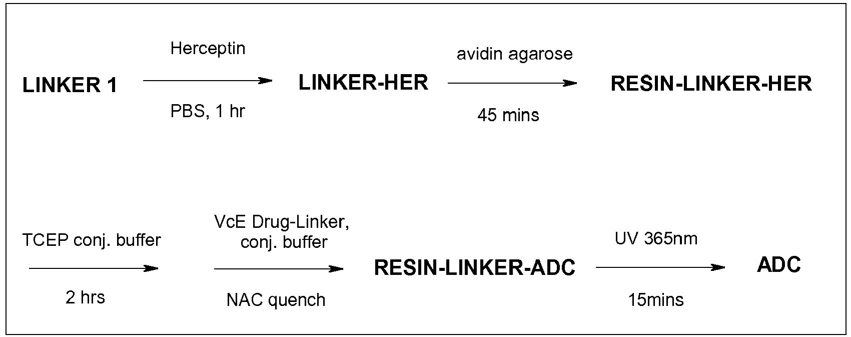

A 1mg/ml solution of Herceptin antibody in PBS (pH7.4) was reacted with Linker 1 then subsequently immobilised to avidin agarose resin as described in Examples 4 and 5.

Resin containing immobilised Linker-Herceptin was transferred to a spin cartridge SigmaPrep (800µl capacity) and washed sequentially with 5 aliquots of conjugation buffer (25mmole Tris containing 1.25mmole EDTA, pH 8.5). The cartridge outlet was capped and 0.5ml of conjugation buffer was added, followed by 2 molar equivalents of the reductant TCEP (tris[2-carboxyethyl]phosphine, 1mmole). The reaction was incubated for 2 hours at ambient temperature with gentle mixing.

After this duration the TCEP and all other superfluous reagents were removed by spinning down the resin and washing the resultant resin bed sequentially with 3 aliquots of conjugation buffer. The resultant Resin-Linker Herceptin complex contained within the spin cartridge was utilised immediately in Example 8.

Example 8: Conjugation of cytotoxic drug linker vcE to activated Resin-Linker-Herceptin complex of Example 7

vcE (vcMMAE) is a maleimide terminated cytotoxic drug linking agent. The cytotoxic warhead is based upon the Dolastatin/Auristatin class of microtubule interacting agents. vcE contains an enzymatic 'self-immolative' linker. The maleimide group facilitates conjugation through reduced thiols on the antibody.

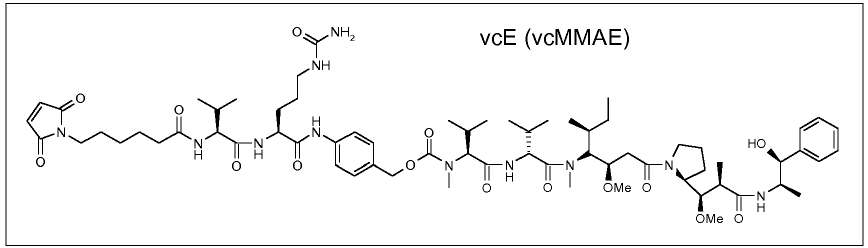

The outlet of the spin cartridge was sealed. A single aliquot of 475µls of conjugation buffer was charged to the damp resin contained within the spin cartridge. A 5 molar equivalent charge of vcE solution (10mmole in DMA, 1.75µl) was added to the resin (total volume of DMA is <5%v/v). The suspension was incubated ambient temperature for 1 hour with gentle mixing. The reaction was quenched by the addition of a charge of with NAC (N-acetyl cysteine, 10 mmol, 1.75µl) and incubated for a further 20 minutes at ambient temperature.

The supernatant was removed by centrifugation. The damp resin bed was then washed sequentially with 2 fresh aliquots of PBS (pH7.4). The damp resin was then transferred to a glass vial and re-mobilised in PBS (pH7.4). The sample was then exposed to UV light (365nm) for up to 15 minutes. The resultant supernatant was then collected and analysed. Verification of photolytic cleavage was confirmed by monitoring UV absorbance. Crude Herceptin-vcE conjugate was analysed by typical chromatographic means using SEC and HIC HPLC. To demonstrate parity the crude Herceptin-vcE obtained by photolysis was directly compared with an analogous Herceptin-vcE conjugate, synthesised by a tradition solution phase methodology. A crude cleavage yield of 38% was calculated by UV at 280nm. HIC determined a drug antibody ratio (DAR) of 1.11 at 280nm.

For comparison, a standard solution phase conjugation using the conditions and equivalents noted above would have targeted a DAR of approximately 3.

### Example 9: Conjugation of cytotoxic drug linker vcE to activated Resin-Linker-Herceptin complex of Example 7 - Improving the DAR

The conjugation process described in Example 7 & 8 was repeated with 4 molar equivalents of TCEP and 8 molar equivalents of vcE. This ratio of components targets a higher DAR species. As expected the HIC elution profile indicated an increase in DAR through the characteristic presence of higher DAR species. Similarly, increasing the TCEP and vcE drug linker equivalents even higher (to 6 molar equivalents of TCEP & 12 molar equivalents of vcE respectively) also demonstrated the same HIC elution pattern to accessing higher DAR species.

### Example 10. Aqueous solution phase reaction of Linker 1 with [Leu]⁵-Enkephalin

The following approach was used to couple a peptide moiety to Linker 1 in solution, then cleavage by photolysis to give the peptide in an unchanged form.

[Leu]⁵-Enkephalin peptide (0.05mg in 150µls PBS, pH7.4) (J. Hughes et al., Nature, 1975, 258, 277-279) was reacted with 3 molar equivalents of Linker 1 (25mmol solution in anhydrous DMF). The reaction was incubated for 1 hour at ambient temperature after which duration an aliquot was removed for RP HPLC and MS analysis. The remaining solution was subjected to UV light exposure (365 nm) for up to 15 minutes, with stirring. The resultant mixture was then analysed by RP HPLC and MS techniques.

In comparison with standards of all reaction components, RP HPLC analysis of the reaction media after UV exposure observed two new entities in the elution profile. MS concluded recovery of the [Leu]⁵-Enkephalin peptide target. Expected mw 555.6, Observed mass 556.1 (M+H)

### Example 11: Synthesis of a (2-hydroxyphenyl)acrylic acid photocleavable linker for traceless cleavage of amines

The (2-hydroxyphenyl)acrylic acid photolabile linker is described as facilitating amino immobilisation and release. Cleavage is based upon UV excitation effecting a trans → cis-alkene isomerisation whereafter intramolecular cyclisation affords a coumarin-like adduct with concomitant release of the amine in an unmodified state.

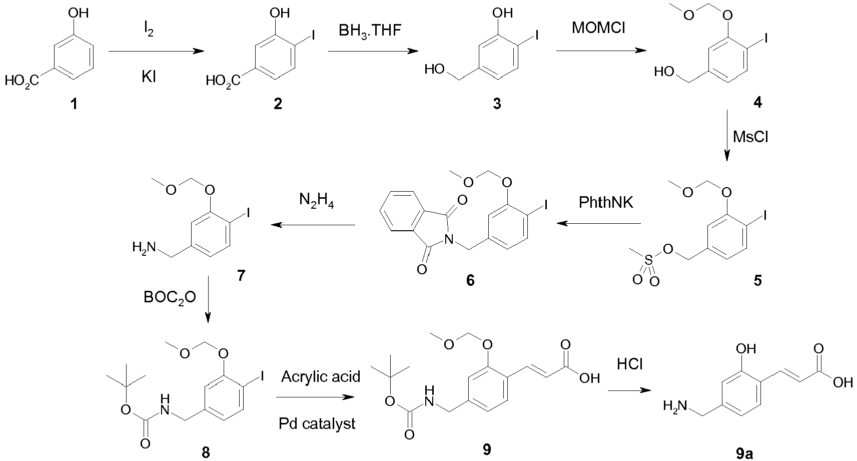

Target acrylic acid compound 9a was synthesised according to the scheme outlined above. Compound 9a was used as the building block for a number of different photo cleavable moieties.

3-Hydroxybenzoic acid 1 (52.0g, 0.376moles) was dissolved in 28% aqueous ammonia (105ml). Iodine (86.4g, 0.340moles) and potassium iodide (68.7g, 0.414moles) in water (415 ml) were added slowly, then the solution stirred for an additional 60 minutes. The mixture was acidified using conc. HCI and the resultant precipitate collected and washed with distilled water. Recrystallisation by dissolution in the minimum of methanol followed by addition of water gave 37.1 g (38 %) of the desired product 4-iodo-3-hydroxybenzoic acid 2 over three crops as a white solid. ¹H NMR (DMSO-d6) 10.66 (s, 1H), 7.79 (d, J = 8.1Hz, 1H), 7.42 (d, J = 1.5Hz, 1H), 7.13 (dd, J = 8.1Hz, 1.5Hz, 1H).

4-lodo-3-hydroxybenzoic acid (18.3g, 69.3mmoles) was dissolved in THF (120ml) and cooled to 0°C. Borane (138ml of a 1M solution in THF) was added drop wise and the solution stirred at room temperature until TLC qualified complete consumption of the carboxylic acid starting material. The reaction was quenched with sodium carbonate (50ml, 1M aq.) then approximately 100ml of THF was removed by rotary evaporation. The resultant reaction mixture was then extracted with EtOAc (3 × 50ml aliquots). The combined organic phases were subsequently washed with brine then dried over anhydrous MgSO₄. Solvent was removed to give the crude 4-iodo-3-hydroxybenzyl alcohol product 3 (15.7g, 90%) which was used without further purification in the next step. ¹H NMR (CDCl₃) 7.55 (d, 1H), 7.30 (br s, 1H), 6.92 (d, 1H), 6.57 (dd, 1H), 4.52 (br d, 2H).

4-lodo-3-hydroxybenzyl alcohol 3 (15.7g crude, 62.8 mmoles) was dissolved in acetone (60ml). Potassium carbonate (19.1g, 138mmoles) was added and the solution cooled to 0°C. Methoxymethyl chloride (5.2ml, 69.1mmol) was added gradually and the reaction mixture stirred at room temperature until TLC analysis qualified complete consumption of the starting material (this required addition of a further 0.3ml of methoxymethyl chloride). Distilled water (100ml) and EtOAc (100ml) were added to the reaction media and the organic phase separated. The aqueous phase was then washed with EtOAc (2 × 100ml aliquots). The combined organic fractions were dried over anhydrous MgSO₄ where after the solvent was removed by rotary evaporation. Purification by column chromatography on silica gel (15 to 20% EtOAc in petrol ether) gave the desired product 4-iodo-3-(methoxymethoxy)benzyl alcohol_(10.3g, 51% over two steps). ¹H NMR (CDCl₃) 7.76 (dd, J = 8.1, 1.8Hz, 1H), 7.10 (d, J = 1.8Hz, 1H), 6.79 (dd, J = 8.1, 1.8Hz, 1H), 5.27 (s, 2H), 4.66 (d, J = 4.5Hz, 2H), 3.53 (s, 3H).

4-lodo-3-(methoxymethoxy)benzyl alcohol 4 (9.41g, 32.0mmoles) was dissolved in dry DCM (100ml). Triethylamine (8.92ml, 64.0mmoles) and DMAP (approx. 50 mg) were added and the solution cooled to 0°C. Mesyl chloride (2.73ml, 35.3mmoles) was added gradually and the resultant reaction mixture stirred at room temperature for 3 hours until TLC qualified consumption of the alcoholic starting material. Distilled water (100ml) was added and the organic phase separated. The aqueous fraction was then extracted with DCM (2 × 50ml aliquots). The combined organic fractions were then washed with brine then dried over anhydrous MgSO₄. Solvent was removed by rotary evaporation. The crude 4-iodo-3-(methoxymethoxy)phenyl methansulfonylmethyl product (8.14g) was used without further purification. ¹H NMR (CDCl₃) 7.82 (d, J = 8.1Hz, 1H), 7.12 (d, J = 1.8Hz, 1H), 6.84 (dd, J = 8.1, 1.8Hz, 1H), 5.28 (s, 2H), 5.19 (s, 2H), 3.53 (s, 3H), 2.98 (s, 3H).

4-lodo-3-(methoxymethoxy)phenyl methansulfonylmethyl 5 (8.14g crude) was mobilised in DMF (50ml). Potassium phthalimide (3.21g, 17.3mmoles) was then added. The resultant reaction mixture was then heated at 70°C under a nitrogen atmosphere for 3 hours after which time the reaction was cooled then diluted with distilled water (200ml). This mixture was extracted with EtOAc (3 × 50ml aliquots) and the combined organic phases washed with brine, dried over anhydrous MgSO₄ and the solvent removed by rotary evaporation. Purification by column chromatography on silica gel (20% EtOAc in petrol ether) gave the product 1-phthalidomethyl-4-lodo-3-(methoxymethoxy)benzene (7.32g, 54% over two steps). ¹H NMR (CDCl₃) 7.80 - 7.60 (two m, 5H), 7.08 (s, 1H), 6.76 (d, 2H), 4.71 (s, 2H), 3.45 (s, 3H).

1-Phthalidomethyl-4-lodo-3-(methoxymethoxy)benzene (3.4g, 8.03mmoles) was dissolved in IMS (75ml). Hydrazine hydrate (1.01 ml, 16.1mmoles) was then added. The resultant solution was heated at 70°C until the phthalhydrazine by product precipitated and qualitative TLC showed no product remaining in solution. This required 6 hours. The solids were removed by filtration then washed with fresh IMS. The collected filtrate was subsequently evaporated to give 2.7g of the crude amino product used in the next step. ¹H NMR (DMSO-d6) 7.67 (d, 1H), 7.11 (s, 1H), 6.78 (d, 1H), 5.27 (s, 2H), 3.67 (s, 2H), 3.45 (s, 3H).

1-Aminomethyl-4-iodo-3-(methoxymethoxy)benzene (2.7g crude) was suspended in THF (75ml) and DMF (2.5ml) and cooled in ice/water. Triethylamine (1.68ml, 12.1mmoles) and DMAP (approx. 25 mg) were added, followed by BOC anhydride (2.63g, 12.1mmoles). The solution was warmed to ambient and after 30 minutes. After stirring at ambient for a further 2 hours qualitative TLC indicated no amine was remaining. Distilled water (100ml) was added and the organic phase separated. The aqueous fraction was extracted with EtOAc (2 × 50ml aliquots) then the combined organic phases were washed with brine then dried over anhydrous MgSO₄. Solvent was then removed by rotary evaporation. Purification by column chromatography on silica gel (20% EtOAc in petrol ether) gave the desired N-Boc protected product (2.89g, 91 % over two steps). ¹H NMR (CDCl₃) 7.72 (d, J = 8.1Hz, 1H), 7.00 (d, J = 1.8Hz, 1H), 6.72 (d, J = 8.1Hz, 1H), 5.25 (s, 2H), 4.27 (br d, J = 6Hz, 2H), 3.53 (s, 3H), 1.48 (s, 9H).

1-*tert*-Butoxycarbonylaminomethyl-3-(methoxymethoxy)-4-iodobenzene (2.89g, 7.35mmoles) was dissolved in DMF (18ml) and degassed with a stream of nitrogen. The solution was subsequently heated to 70°C with continued degassing. K₂CO₃ (1.52g, 11.0mmoles) in water (18ml) was added to the reaction mixture. After a further 10 minutes degassing, Pd(OAc)₂ catalyst (82mg, 0.36mmoles) and acrylic acid (0.75ml, 11.0mmoles) were charged. The resultant mixture was heated under nitrogen. After 10 minutes a black suspension formed. After 30 minutes the reaction was deemed complete by TLC. Distilled water (50ml) was added and the pH adjusted to 5 using 2M HCI. The aqueous fraction was extracted with EtOAc (2 × 50ml aliquots) and the combined organic fractions dried over anhydrous MgSO₄ and solvent evaporated. Crude product was purified using column chromatography (pet. Ether/10% EtOAc/20 % acetone) to give the desired alkene product (1.60g, 65%). ¹H NMR (CDCl₃) 8.09 (d, J = 16.2Hz, 1H), 7.53 (d, J = 8.1Hz, 1H), 7.09 (s, 1H), 6.97 (d, J = 8.1, 1H), 6.52 (d, J = 16.2Hz, 1H), 5.27 (s, 2H), 4.32 (br d, J = 5.7Hz, 2H), 3.51 (s, 3H), 1.48 (s, 9H).

4-*tert*-Butoxycarbonylaminomethyl-2-(methoxymethoxy)phenyl acrylic acid (0.6g, 1.78mmoles) was stirred in 2M HCI/dioxane (12ml) at room temperature. A clear solution was formed initially followed by a white solid precipitate. TLC and LC-MS analysis demonstrated complete consumption of N- Boc-protected starting materials after 18 hours. The solvent was removed to give the desired free amine product as the HCI salt (0.395g, 96% for HCl salt). ¹H NMR (DMSO-d6) 10.47 (s, 1H), 8.25 (br s, 2H), 7.80 (d, J = 16.2Hz, 1H), 7.64 (d, J = 7.8Hz, 1H), 6.92 & 6.86 (unresolved s overlaid on d, 2H), 6.54 (d, J = 16.2Hz, 1H), 4.32 (br apparent q, d, J = 5.7Hz, 2H).

### Example 12: Synthesis of a model system to demonstrate UV cleavage of new coumarin-like photolabile linker

In this example we have utilised the 4-aminomethyl-2-hydroxyl phenyl acrylic acid linker afforded in Example 11 and derivatised to an amine reactive intermediate via N-hydroxysuccimidyl activation. This amine reactive intermediate was then subsequently reacted with an organic amino naphyl compound to afford a covalently linked Linker-naphyl complex. After isolation and purification the model complex was subjected to UV exposure to release the amino naphyl compound in a traceless manner. The outline of the synthesis and process is shown in the schematic below.

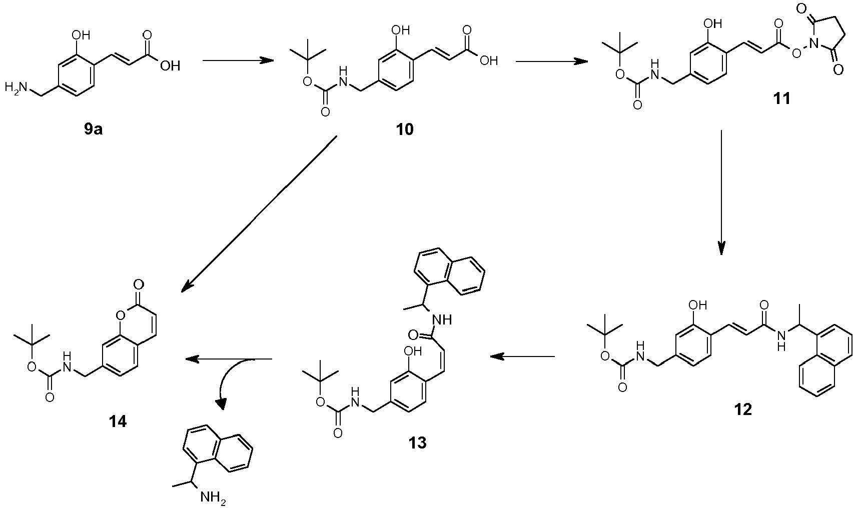

Selective N-Boc protection of 4-aminomethyl-2-hydroxyphenyl acrylic acid linker 9a was performed to facilitate an organic soluble linker to test as a model system. 4-Aminomethyl-2-hydroxyphenyl acrylic acid (85mg, 0.37mmoles) was dissolved in DMF. An aliquot of Et₃N (0.78ml of a 10%v/v solution in DMF, 0.56mmoles) was charged followed by Boc anhydride (0.89ml of a 10%w/v solution in DMF, 0.41 mmoles). The resultant solution was stirred at ambient temperature overnight then concentrated by rotary evaporation. Following purification by column chromatography in 10%v/v MeOH in DCM the target N-protected 4-*tert*-butoxycarbonylaminomethyl-2-hydroxyphenyl acrylic acid linker (116mg, compound 10).

The intermediate 4-*tert*-butoxycarbonylaminomethyl-2-hydroxyphenyl acrylic acid (32mg, 0.11 mmoles) was dissolved in DMF (0.2ml). A single aliquot of DIC (0.17ml of a 10 %v/v solution in DMF, 0.11 mmoles) was added followed by N-hydroxysuccinimide (12.6mg, 0.11 mmoles). The mixture was then stirred at ambient temperature overnight. LC-MS analysis indicated a small portion of starting material was remaining hence a further portion of DIC was added (0.17ml of a 10%v/v solution in DMF, 0.11 mmoles). After 4 hours the reaction was qualified as complete by LC-MS analysis. Crude N-(4-tert-butoxycarbonylaminomethyl-2-hydroxyphenyl acryloxy) hydroxysuccinimide ester was purified by column chromatography in 1%v/v MeOH in DCM (29mg).

N-(4-*tert*-butoxycarbonylaminomethyl-2-hydroxyphenyl acryloxy) hydroxysuccinimide ester (38mg, 0.097mmoles) was dissolved in DCM (1ml) and DMF (0.1ml). 1-(1-Naphthyl)-ethylamine (16.7mg, 0.097mmoles) was added and the solution quickly became yellow. After stirring at ambient temperature overnight, LC-MS analysis indiacted a new peak for the product. Solvents were removed in vacuo. The resultant residue was purified by column chromatography (1%v/v MeOH in DCM) to give the desired product (18mg, 42%). HPLC-MS 447.2 (M+H)+; ¹H NMR (CDCl₃) 8.10 (1H, d, naphthyl H), 7.85 (1H, d, alkene H), 7.80 (1H, d, naphthyl H), 7,75 (1H, d, naphthyl H), 7.5 - 7.3 (3H, m, naphthyl H), 7.20 (1H, d, aryl H), 6.70 (1H, s, aryl H), 6.65 (1H, d, aryl H), 6.40 (d, 1H, alkene H), 6.00 (1H, q, CH), 5.90 (1H, d, naphthyl H) 4.10 (2H, d, CH2), 1.65 (3H, d, CH3), 1.35 (9H, s, t-Bu)

### Example 13: Demonstration of UV cleavage for new coumarin-like photolabile linker

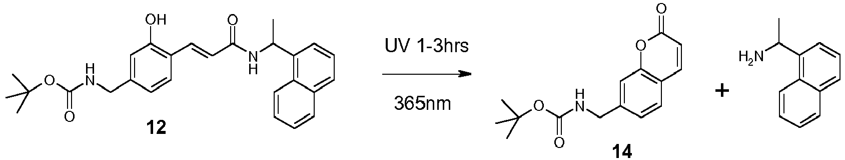

A solution of the model complex N-(4-tert-butoxycarbonylaminomethyl-2-hydroxyphenyl acryloxy)-(1-naphthyl)ethylamine (2mg, 4.46µmoles) in DCM was placed into a glass vial. A time course study was undertaken over a period of 3.5 hours whereby the solution of Linker-naphthylamine was subjected to UV exposure at 365nm. At regular intervals over the course of the study the reaction solution was sampled and analysed by RP HPLC and NMR techniques to qualify and quantify the release of 1-(1-naphthyl)ethylamine. NMR was found to be a useful technique in showing the photocatalysed conversion of trans → cis isomer in the linker prior to release of the amine target. Our analysis indicated that trans → cis conversion (Compound 13) was complete within 2 hours. Subsequent cyclisation and release of amine is slower with 34% conversion quantified after 24 hours.

The identity of the released amine was confirmed as 1-(1-naphthyl)ethylamine using RP-HPLC and MS analysis. Best results were seen using a DCM media in combination with catalytic acetic acid (0.02molar equivalents). Exposure of the Linker-naphthyl complex to UV at 365nm for 3.5 hours instigates cleavage affording 93% conversion to the desired free amine. Identity of the cleaved amine was verified by RP-HPLC and MS analysis. A minor bi-product (Compound 12) was clearly seen by MS and RP-HPLC.

These studies confirm that the new linker can react with an amine in non-aqueous media and undergo subsequent reversible cleavage initiated by UV photolysis releasing the molecule in an unmodified fashion.

### Example 14: Synthesis of a Biotin-PEG-coumarin-like photolabile linker

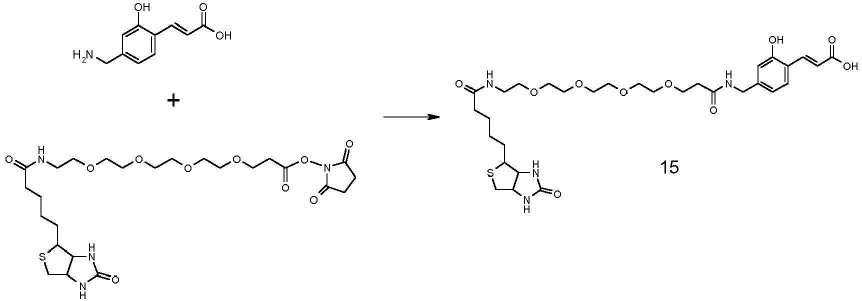

Attachment of a biotin-PEG4 chain was performed using EZ-Link NHS ester-PEG4-Biotin (Pierce) and 4-aminomethyl-2-hydroxylphenyl acrylic acid linker.HCl salt (Compound 9a, Example 11). 4-Aminomethyl-2-hydroxyphenyl acrylic acid. HCI salt (62mg, 0.29mmoles) was dissolved in dry DMF (1ml). Diisopropylethylamine (47µl, 0.29mmoles) was charged which resulted in the formation of a precipitate. EZ-Link NHS ester-PEG4-Biotin (159mg, 0.29mmoles) in DCM (1ml) was added and the resultant reaction mixture stirred at ambient temperature overnight, resulting in a clear pale yellow solution. Following rotary evaporation the resultant residue was purified by flash chromatography on silica gel (100% DCM then 10%v/v MeOH in DCM). The desired product Biotin-PEG4-4-aminomethyl-2-hydroxylphenyl acrylic acid was obtained as a clear sticky solid (120mg, 62%, Compound 15). Verification of the product identity was confirmed using LC-MS analysis. LC-MS Rt 1.44min, Expected mw 666.8, Experimental mw 667.4 (M+H). 1H NMR (DMSO-d6, 300 MHz) 10.20 (s, 1 H, phenol OH), 8.35 (br t, 1 H, NH), 7.82 (br t, 1 H, NH), 7.79 (d, J = 16 Hz, 1 H, alkene H), 7.50 (d, J = 7 Hz, 1 H, aryl H), 6.79 (s, 1 H, aryl H), 6.73 (d, J = 7 Hz, 1 H, aryl H), 6.49 (d, J = 16 Hz, 1 H, alkene H), 6.41 (s, 1 H, biotin NH), 6.23 (s, 1 H, biotin NH), 4.32 (m, 1 H, biotin CH), 4.20 (d, J = 7 Hz, 2 H, benzyl CH2), 4.15 (m, 1 H, biotin CH), 3.63 (t, J = 6 Hz, 2 H, CH2), 3.49 (br s, 6 H, PEG CH2), 3.39 (t, J = 8 Hz, 2 H, CH2), 3.2-3.16 (m, 6 H, CH2), 3.10 (m, 1 H, biotin CH), 2.82 (dd, J = 18 Hz, 6 Hz, 1 H, biotin CH), 2.59 (d, J = 18 Hz, 1 H, biotin CH), 2.40 (t, J = 6 Hz, 2 H, CH2), 2.06 (t, J = 6 Hz, 2 H, CH2), 1.65-1.15 (m, 8 H, CH2).

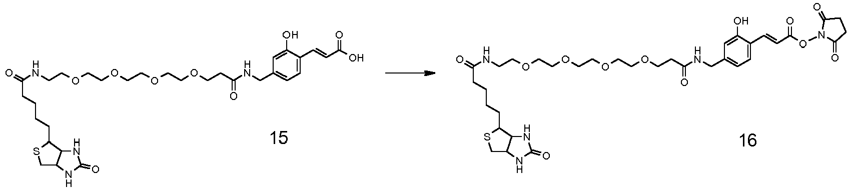

Biotin-PEG4-4-aminomethyl-2-hydroxylphenyl acrylic acid (20mg, 0.03mmoles) was dissolved in dry DMF (0.5ml). A single charge of DIC (10µl, 0.063mmoles) was added at ambient temperature. After stirring for 10 minutes, N-hydroxysuccinimide (4.1mg, 0.036mmoles) was added and the resultant reaction mixture stirred at ambient temperature for 18 hours. LC-MS analysis of the reaction mixture indicated consumption of the free acid starting material. The desired product was precipitated by the addition of dry diethyl ether (3ml) to give a clear sticky solid. Precipitated solid was washed with sonication using two further 3ml aliquots of diethyl ether. The solid was then filtered then dried under vacuum to give the desired product Biotin-PEG4-4-aminomethyl-2-hydroxylphenyl acryl hydroxysuccinimide ester as a white hygroscopic solid (15.7mg, 69%, Compound 16). LC-MS Rt 1.53min, Expected mw 763.9, Experimental mw 764.3 (M+H). 1H NMR (DMSO-d6, 300 MHz) 10.60 (s, 1 H, phenol OH), 8.42 (br t, J = 6 Hz, 1 H, NH), 8.08 (d, J = 15 Hz, 1 H, alkene H), 7.95 (s, 1 H, aryl H), 7.86 (br t, J = 6 Hz, 1 H, NH), 7.69 (d, J = 8 Hz, 1 H, aryl H), 6.88 (d, J = 15 Hz, 1 H, alkene H), 6.78 (d, J = 6 Hz, 1 H, aryl H), 6.44 (s, 1 H, biotin NH), 6.38 (s, 1 H, biotin NH), 4.32 (m, 1 H, biotin CH), 4.23 (d, J = 6 Hz, 2 H, benzyl CH2), 4.14 (m, 1 H, biotin CH), 3.64 (t, J = 6 Hz, 2 H, CH2), 3.50 (br s, 6 H, PEG CH2), 3.41 (m, 4 H, CH2), 3.19 (m, 2 H, CH2), 3.10 (m, 1 H, biotin CH), 2.9-2.7 (m, 7 H, 1 biotin CH, 4 succinimide CH2 and 2 CH2), 2.57 (m, 1 H, biotin CH), 2.40 (t, J = 6 Hz, 2 H, CH2), 2.06 (t, J = 6 Hz, 2 H, CH2), 1.65-1.15 (m, 8 H, CH2).

### Example 15: Solution phase coupling of Biotin-PEG4-4-aminomethyl-2 hydroxylphenyl acryl hydroxysuccinimide ester to [Leu]⁵-Enkephalin peptide

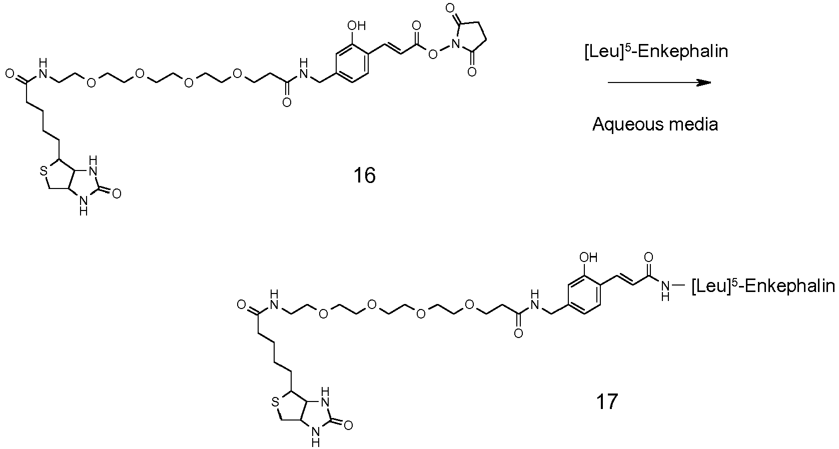

The coupling reaction above can be carried out in water, PBS (pH7.4) and 0.1M NaHCO₃. By example, the model N-amino terminated peptide [Leu]⁵-enkephalin (80µls of a standard solution of the peptide in PBS (pH7.4, 6mg/ml 0.48mg, 0.86µmole) was diluted to 1ml with water and reacted with Biotin-PEG4-4-aminomethyl-2-hydroxylphenyl acryl hydroxysuccinimide ester in DMF 105µl, 2.62µmoles. The reaction mixture was incubated for 1 hour at ambient temperature with gentle stirring. Verification of coupling was confirmed by RP-HPLC consumption of the amino peptide and hydrolysed excess linker. LC-MS analysis corrected identified the target product Biotin-PEG4-4-aminomethyl-2-hydroxylphenyl acryl-[Leu]⁵-enkephalin (Compound 17). Expected mw 1204.4, Experimental mw 1204.7(M+H).

### Example 16: Demonstration of UV cleavage for Biotin-PEG4-4-aminomethyl-2-hydroxylphenyl acryl-[Leu]⁵-enkephalin peptide

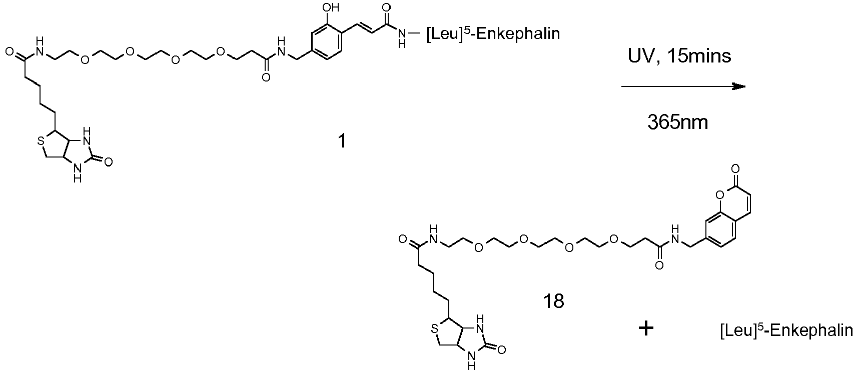

Biotin-PEG4-4-aminomethyl-2-hydroxylphenyl acryl-[Leu]⁵-enkephalin peptide complex obtained in Example 15 (450 µls)was subjected to UV exposure for 15 minutes at 365nm. A sample of the reaction media was then removed and analysed by RP-HPLC and LC-MS. After photolysis 3 main peaks were seen in the LCMS elution profile, corresponding to released, unmodified [Leu]⁵-enkephalin peptide, UV bi-product from linker (Compound 18) and an amount of starting material Biotin-PEG4-4-aminomethyl-2-hydroxylphenyl acryl-[Leu]⁵-enkephalin.

RP-HPLC controls were undertaken to confirm the identity of the cleaved [Leu]⁵-enkephalin peptide and also to qualify that the peptide had been unmodified by the UV exposure. Furthermore, the crude reaction mixture was spiked with increasing amounts of a standard of [Leu]⁵-enkephalin which responded with an increase in peak area of the peak corresponding to [Leu]⁵-enkephalin peptide. As a further control a standard of the [Leu]⁵-enkephalin peptide was exposed under identical UV conditions. RP-HPLC analysis demonstrated no degradation of the peptide under these conditions. Similarly, to obtain a standard for Compound 18 we subjected hydrolysed (free acid) linker to identical UV exposure conditions (15 minutes, 365nm) and obtained the desired bi-product. Co-injection of this material with the crude cleavage mixture confirmed identity. To qualify identity all components were analysed by LC-MS. [Leu]⁵-enkephalin peptide expected mw 555.6, Experimental mw 556.3 (M+H); Biotin-PEG4-4-aminomethyl-coumarin adduct expected mw 648.8, Experimental mw 671.3 (M+23).

These studies confirm that the new coumarin-like linker can react with an amino based proteinaceous moiety in aqueous conditions and upon selective photolysis release the proteinaceous moiety in an unmodified form. The UV cleavage mechanism would appear to be traceless and non-invasive.

## Claims

1. A method of synthesising a biomolecule-drug-conjugate, the method comprising:
(i) either:
(a) contacting a biomolecule having a pendent -NRH group with a support that is functionalised with a photocleavable group under conditions suitable to covalently bond the biomolecule to the photocleavable group; or
(b) contacting a biomolecule having a pendent -NRH group with a photocleavable group under conditions suitable to covalently bond the biomolecule to the photocleavable group to produce a photocleavable group-biomolecule conjugate, and then immobilising the photocleavable group-biomolecule conjugate to a support;
to provide an immobilised photocleavable group-biomolecule conjugate: 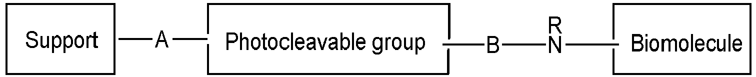
(ii) optionally contacting the immobilised photocleavable group-biomolecule conjugate with a chemical modification agent or activating agent to provide an immobilised photocleavable group-biomolecule conjugate that is chemically modified or activated at the biomolecule portion;
(iii) contacting the immobilised photocleavable group-biomolecule conjugate of step (i) or the chemically modified or activated immobilised photocleavable group-biomolecule conjugate of step (ii) with a drug component to form an immobilised photocleavable group-biomolecule-drug-conjugate: 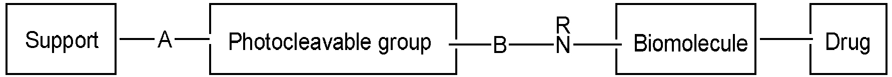
(iv) irradiating the immobilised photocleavable group-biomolecule-drug-conjugate of step (iii) with radiation thereby releasing the biomolecule-drug-conjugate 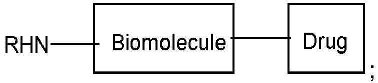 wherein:
-A- is absent or is a spacer group;
-B- is absent or is a spacer group;
'Photocleavable group' comprises any residue that is able to cleave upon exposure to radiation to allow release of the biomolecule-drug-conjugate;
R is selected from the group consisting of: H, C₁-C₈ alkyl, C₂-C₈ alkenyl, C₂-C₈ alkynyl, C₁-C₈ haloalkyl, -(CR^{a}R^{a})ₙ-C₃-C₆ cycloalkyl, -(CR^{a}R^{a})ₙ-C₃-C₆ heterocycloalkyl, -(CR^{a}R^{a})ₙ-C₃-C₆ halocycloalkyl, -(CR^{a}R^{a})ₙ-aryl, and -(CR^{a}R^{a})ₙ-heteroaryl;
R^{a} is independently at each occurrence selected from: H, Me, CF₃ and F;
n is an integer independently selected at each occurrence from 0, 1, 2 and 3; and
'Drug' is a residue of a drug molecule selected from the group consisting of tubulin inhibitors and DNA interacting agents.

2. The method of claim 1, wherein step (i) comprises incubating for a period of time of from about 10 minutes to about 1 day at a temperature of from about 10°C to about 30°C.

3. The method of any preceding claim, wherein step (ii) is mandatory and comprises reducing the biomolecule; optionally
wherein step (ii) comprises reducing the biomolecule with tris(2-carboxyethyl)phosphine (TCEP).

4. The method of any preceding claim, wherein step (iii) comprises contacting with a drug component in a buffer solution.

5. The method of any preceding claim, wherein step (iv) comprises irradiating with UV light having a wavelength of from about 320 nm to about 400 nm, preferably a wavelength of about 365 nm.

6. The method of any preceding claim, wherein the support is a bead; and/or is agarose based.

7. The method of any preceding claim, wherein the biomolecule is selected from the group comprising: antibodies, antibody fragments, modified antibodies, enzymes, proteins, peptides, polypeptides, modified peptides, peptide nucleic acids (PNAs), metalloproteins, peptide-oligonucleotide hybrids, amino acids, non-naturally occurring amino acids, diamino acids, synthetic amino acids, oligonucleotides, modified oligonucleotides, nucleotides, nucleosides, purines, pyrimidines, oligosaccharides, polysaccharide, disaccharides, monosaccharides, amino sugars, lipids, phospholipids, glycolipids, sterols, vitamins, hormones, steroids, neurotransmitters, carbohydrates, sugars, viruses, cells, active pharmaceutical ingredients (APIs), and precursor compounds or a derivatives of any of these.

8. The method of any of claims 1 to 7, wherein the photocleavable group is a 2-nitrophenzyl group of Formula IIa: 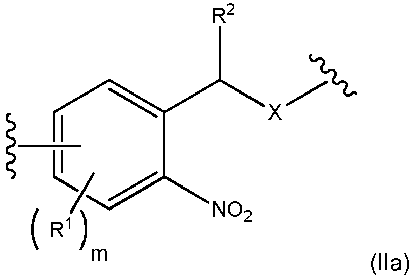 such that the immobilised photocleavable group-biomolecule conjugate has a structure: 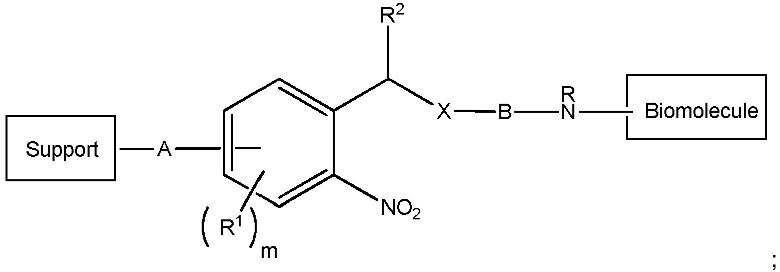 wherein:
each R¹ is a group independently selected from the group consisting of: substituted or unsubstituted C₁-C₄ alkyl, substituted or unsubstituted C₁-C₄ haloalkyl, substituted or unsubstituted C₁-C₄ alkoxyl and substituted or unsubstituted C₁-C₄ haloalkoxyl;
R² is selected from the group consisting of: H, substituted or unsubstituted C₁₋₄ alkyl and substituted or unsubstituted C₁-C₄ haloalkyl;
X is a group selected from the group consisting of: -O- and -S-; and
m is an integer of 0 to 3;
wherein each of the aforementioned alkyl, haloalkyl, alkoxyl and haloalkoxyl groups is optionally substituted, where chemically possible, by 1 to 5 substituents which are each independently at each occurrence selected from the group consisting of: oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a} CO₂R^{a} C(O)R^{a}, CONR^{a}R^{a}, C₁-C₄-alkyl, C₂-C₄-alkenyl, C₂-C₄-alkynyl, C₁-C₄ haloalkyl, CR^{b}R^{b}NR^{a}R^{a}, and =CR^{b}CR^{b}R^{b}NR^{a}R^{a};
wherein R^{a} is independently at each occurrence selected from H, C₁-C₄ alkyl and C₁-C₄ haloalkyl; and R^{b} is independently at each occurrence selected from H, halogen, C₁-C₄ alkyl and C₁-C₄ haloalkyl; optionally
wherein R¹ is methoxy; m is 1; the R¹ substituent is para to the -NO₂ substituent; R² is methyl; and X is -O-.

9. The method of any of claims 1 to 7, wherein the photocleavable group is a benzoin group of Formula IIb: 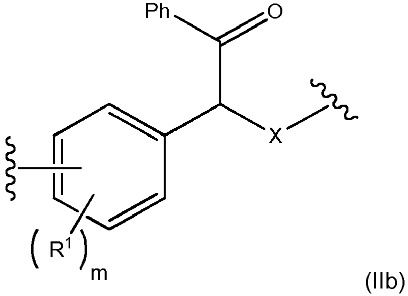 such that the immobilised photocleavable group-biomolecule conjugate has a structure: 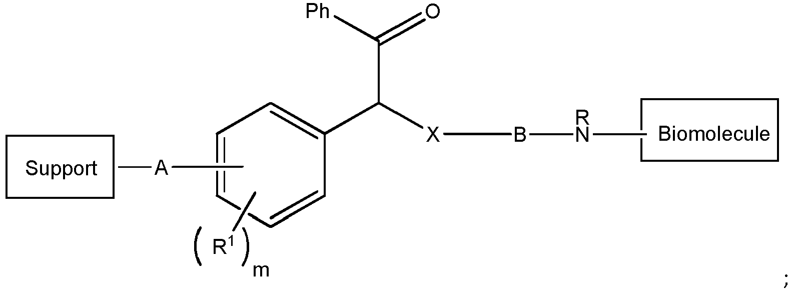 wherein:
each R¹ is a group independently selected from the group consisting of: OH, OR' and OCOR'; wherein R' is selected from the group consisting of: substituted or unsubstituted C₁₋₄ alkyl, substituted or unsubstituted C₁-C₄ haloalkyl, substituted or unsubstituted aryl and substituted or unsubstituted heteroaryl;
X is a group selected from the group consisting of: -O- and -S-; and
m is an integer of 0 to 4;
wherein each of the aforementioned alkyl, haloalkyl, aryl and heteroaryl groups is optionally substituted, where chemically possible, by 1 to 5 substituents which are each independently at each occurrence selected from the group consisting of: oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, C₁-C₄-alkyl, C₂-C₄-alkenyl, C₂-C₄-alkynyl, C₁-C₄ haloalkyl, CR^{b}R^{b}NR^{a}R^{a}, and =CR^{b}CR^{b}R^{b}NR^{a}R^{a};
wherein R^{a} is independently at each occurrence selected from H, C₁-C₄ alkyl and C₁-C₄ haloalkyl; and R^{b} is independently at each occurrence selected from H, halogen, C₁-C₄ alkyl and C₁-C₄ haloalkyl; optionally
wherein R¹ is methoxy; m is 1; the R¹ substituent is meta to the fixed substituent of the benzoin group of Formula IIb; and X is -O-.

10. The method of any of claims 1 to 7, wherein the photocleavable group is a phenacyl group of Formula IIc: 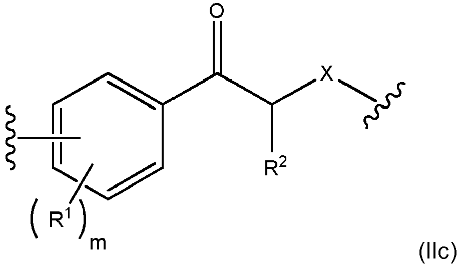 such that the immobilised photocleavable group-biomolecule conjugate has a structure: 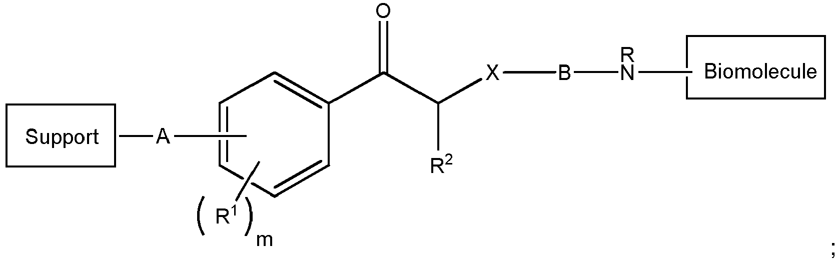 wherein:
each R¹ is a group independently selected from the group consisting of: OH, OR' and OCOR'; wherein R' is selected from the group consisting of: substituted or unsubstituted C₁₋₄ alkyl, substituted or unsubstituted C₁-C₄ haloalkyl, substituted or unsubstituted aryl and substituted or unsubstituted heteroaryl;
R² is selected from the group consisting of: H, C₁₋₄ alkyl and C₁-C₄ haloalkyl;
X is a group selected from the group consisting of: -O- and -S-; and
m is an integer of 0 to 4;
wherein each of the aforementioned alkyl, haloalkyl, aryl and heteroaryl groups is optionally substituted, where chemically possible, by 1 to 5 substituents which are each independently at each occurrence selected from the group consisting of: oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, C₁-C₄-alkyl, C₂-C₄-alkenyl, C₂-C₄-alkynyl, C₁-C₄ haloalkyl, CR^{b}R^{b}NR^{a}R^{a}, and =CR^{b}CR^{b}R^{b}NR^{a}R^{a};
wherein R^{a} is independently at each occurrence selected from H, C₁-C₄ alkyl and C₁-C₄ haloalkyl; and R^{b} is independently at each occurrence selected from H, halogen, C₁-C₄ alkyl and C₁-C₄ haloalkyl; optionally
wherein R¹ is methoxy; m is 1; the R¹ substituent is ortho or para to the fixed substituent of the benzoin group of Formula IIc; R² is H; and X is -O-.

11. The method of any of claims 1 to 7, wherein the photocleavable group is a 4-hydroxy phenacyl group of Formula IId: 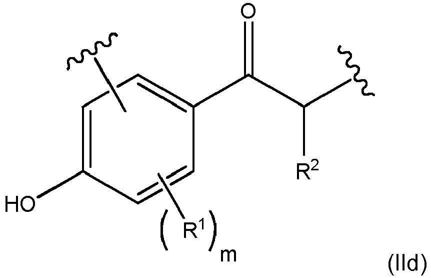 such that the immobilised photocleavable group-biomolecule conjugate has a structure: 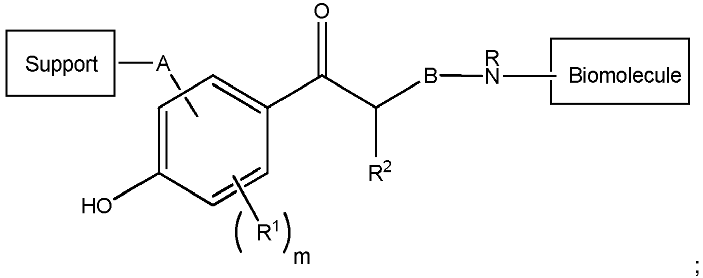 wherein:
each R¹ is a group independently selected from the group consisting of: OH, OR' and OCOR'; wherein R' is selected from the group consisting of: substituted or
unsubstituted C₁₋₄ alkyl, substituted or unsubstituted C₁-C₄ haloalkyl, substituted or unsubstituted aryl and substituted or unsubstituted heteroaryl;
R² is selected from the group consisting of: C₁₋₄ alkyl and C₁-C₄ haloalkyl; and
m is an integer of 0 to 3;
wherein each of the aforementioned alkyl, haloalkyl, aryl and heteroaryl groups is optionally substituted, where chemically possible, by 1 to 5 substituents which are each independently at each occurrence selected from the group consisting of: oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, C₁-C₄-alkyl, C₂-C₄-alkenyl, C₂-C₄-alkynyl, C₁-C₄ haloalkyl, CR^{b}R^{b}NR^{a}R^{a}, and =CR^{b}CR^{b}R^{b}NR^{a}R^{a};
wherein R^{a} is independently at each occurrence selected from H, C₁-C₄ alkyl and C₁-C₄ haloalkyl; and R^{b} is independently at each occurrence selected from H, halogen, C₁-C₄ alkyl and C₁-C₄ haloalkyl; optionally
wherein m is 0; and R² is H.

12. The method of any of claims 1 to 7, wherein the photocleavable group is a (2-hydroxyphenyl)acrylic acid group of Formula IIe: 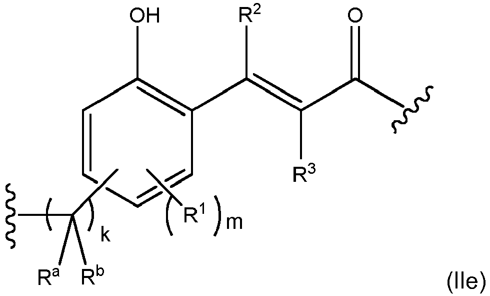 such that the immobilised photocleavable group-biomolecule conjugate has a structure: 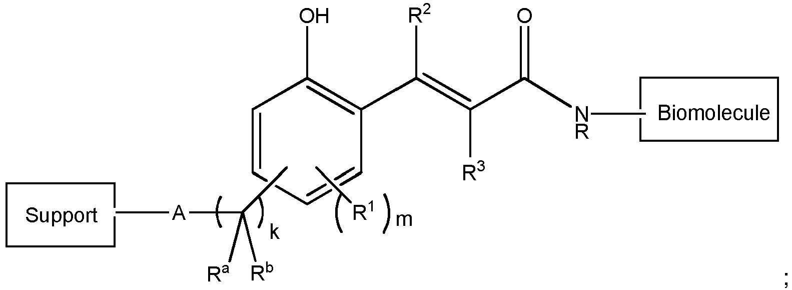 wherein:
each R¹ is a group independently selected from the group consisting of: OH, OR' and OCOR'; wherein R' is selected from the group consisting of: substituted or
unsubstituted C₁₋₄ alkyl, substituted or unsubstituted C₁-C₄ haloalkyl, substituted or unsubstituted aryl and substituted or unsubstituted heteroaryl;
R² and R³ are each independently selected from the group consisting of: H, C₁₋₄ alkyl and C₁-C₄ haloalkyl;
R^{a} and R^{b} are each independently selected from the group consisting of: H, C₁₋₄ alkyl and C₁-C₄ haloalkyl;
k is an integer of 0 to 3; and
m is an integer of 0 to 3;
wherein each of the aforementioned alkyl, haloalkyl, aryl and heteroaryl groups is optionally substituted, where chemically possible, by 1 to 5 substituents which are each independently at each occurrence selected from the group consisting of: oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a}, CO₂R^{a} C(O)R^{a}, CONR^{a}R^{a}, C₁-C₄-alkyl, C₂-C₄-alkenyl, C₂-C₄-alkynyl, C₁-C₄ haloalkyl, CR^{b}R^{b}NR^{a}R^{a}, and =CR^{b}CR^{b}R^{b}NR^{a}R^{a};
wherein R^{a} is independently at each occurrence selected from H, C₁-C₄ alkyl and C₁-C₄ haloalkyl; and R^{b} is independently at each occurrence selected from H, halogen, C₁-C₄ alkyl and C₁-C₄ haloalkyl; optionally
wherein m is 0; k is 0 or 1; R² is H; R³ is H; R^{a} is H; R^{b} is H.

13. The method of any preceding claim, wherein B is -C(=O)- or absent.

14. The method of any preceding claim, wherein R is H.

## Patentansprüche

1. Verfahren zur Synthese eines Biomolekül-Arzneimittel-Konjugats, wobei das Verfahren Folgendes umfasst:
(i) entweder:
(a) Inkontaktbringen eines Biomoleküls, das eine anhängige -NRH-Gruppe aufweist, mit einem Träger, der mit einer photospaltbaren Gruppe funktionalisiert ist, unter Bedingungen, die geeignet sind, das Biomolekül kovalent an die photospaltbare Gruppe zu binden; oder
(b) Inkontaktbringen eines Biomoleküls, das eine anhängige -NRH-Gruppe aufweist, mit einer photospaltbaren Gruppe unter Bedingungen, die geeignet sind, das Biomolekül kovalent an die photospaltbare Gruppe zu binden, um ein photospaltbares Gruppen-Biomolekül-Konjugat zu erzeugen, und dann Immobilisieren des photospaltbaren Gruppen-Biomolekül-Konjugats an einen Träger;
um ein immobilisiertes, photospaltbares Gruppen-Biomolekül-Konjugat bereitzustellen: 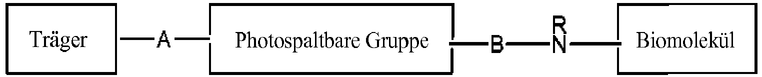
(ii) optional Inkontaktbringen des immobilisierten, photospaltbaren Gruppen-Biomolekül-Konjugats mit einem chemischen Modifizierungsmittel oder Aktivierungsmittel, um ein immobilisiertes, photospaltbares Gruppen-Biomolekül-Konjugat bereitzustellen, das chemisch modifiziert oder an dem Biomolekülabschnitt aktiviert ist;
(iii) Inkontaktbringen des immobilisierten photospaltbaren Gruppen-Biomolekül-Konjugats von Schritt (i) oder des chemisch modifizierten oder aktivierten immobilisierten photospaltbaren Gruppen-Biomolekül-Konjugats von Schritt (ii) mit einer Arzneimittelkomponente, um ein immobilisiertes photospaltbares Gruppen-Biomolekül-Arzneimittel-Konjugat zu bilden: 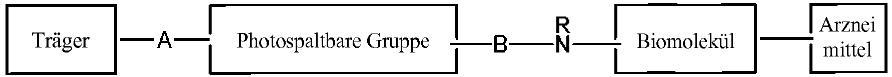
(iv) Bestrahlen des immobilisierten, photospaltbaren Gruppen-Biomolekül-Arzneimittel-Konjugats von Schritt (iii) mit Strahlung, wodurch das Biomolekül-Arzneimittel-Konjugat freigesetzt wird 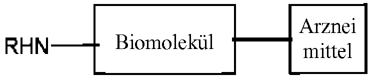 wobei:
-A- fehlt oder eine Spacer-Gruppe ist;
-B- fehlt oder eine Spacer-Gruppe ist;
,photospaltbare Gruppe' einen beliebigen Rest umfasst, der sich bei Aussetzung an Strahlung spalten kann, um eine Freisetzung des Biomolekül-Arzneimittel-Konjugats zu ermöglichen;
R ausgewählt ist aus der Gruppe bestehend aus: H, C₁-C₈-Alkyl, C₂-C₈-Alkenyl, C₂-C₈-Alkynyl, C₁-C₈-Haloalkyl, -(CR^{a}R^{a})ₙ-C₃-C₆-Cycloalkyl, -(CR^{a}R^{a})ₙ-C₃-C₆-Heterocycloalkyl, -(CR^{a}R^{a})ₙ-C₃-C₆-Halocycloalkyl, -(CR^{a}R^{a})ₙ-Aryl und -(CR^{a}R^{a})ₙ-Heteroaryl;
R^{a} bei jedem Vorkommen unabhängig ausgewählt ist aus: H, Me, CF₃ und F;
n eine Ganzzahl ist, die unabhängig bei jedem Vorkommen ausgewählt ist aus 0, 1, 2 und 3; und
'Arzneimittel' ein Rest eines Arzneimittelmoleküls ist, ausgewählt aus der Gruppe bestehend aus Tubulin-Inhibitoren und mit DNA interagierenden Mitteln.

2. Verfahren nach Anspruch 1, wobei Schritt (i) ein Inkubieren über einen Zeitraum von etwa 10 Minuten bis etwa 1 Tag bei einer Temperatur von etwa 10 °C bis etwa 30 °C umfasst.

3. Verfahren nach einem vorherigen Anspruch, wobei Schritt (ii) obligatorisch ist und ein Reduzieren des Biomoleküls umfasst; optional
wobei Schritt (ii) ein Reduzieren des Biomoleküls mit tris(2-Carboxyethyl)phosphin (TCEP) umfasst.

4. Verfahren nach einem vorherigen Anspruch, wobei Schritt (iii) ein Inkontaktbringen mit einer Arzneimittelkomponente in einer Pufferlösung umfasst.

5. Verfahren nach einem vorherigen Anspruch, wobei Schritt (iv) ein Bestrahlen mit UV-Licht mit einer Wellenlänge von etwa 320 nm bis etwa 400 nm, vorzugsweise einer Wellenlänge von etwa 365 nm, umfasst.

6. Verfahren nach einem vorherigen Anspruch, wobei der Träger ein Kügelchen ist; und/oder auf Agarose basiert.

7. Verfahren nach einem vorherigen Anspruch, wobei das Biomolekül ausgewählt ist aus der Gruppe, die Folgendes umfasst: Antikörper, Antikörperfragmente, modifizierte Antikörper, Enzyme, Proteine, Peptide, Polypeptide, modifizierte Peptide, Peptidnukleinsäuren (PNAs), Metalloproteine, Peptid-Oligonukleotidhybride, Aminosäuren, nicht natürlich vorkommende Aminosäuren, Diaminosäuren, synthetische Aminosäuren, Oligonukleotide, modifizierte Oligonukleotide, Nukleotide, Nukleoside, Purine, Pyrimidine, Oligosaccharide, Polysaccharide, Disaccharide, Monosaccharide, Aminozucker, Lipide, Phospholipide, Glycolipide, Sterole, Vitamine, Hormone, Steroide, Neurotransmitter, Kohlenhydrate, Zucker, Viren, Zellen, Wirkstoffe (active pharmaceutical ingredients, APIs) und Vorläuferverbindungen oder ein Derivat von beliebigen dieser.

8. Verfahren nach einem der Ansprüche 1 bis 7, wobei die photospaltbare Gruppe eine 2-Nitrophenzyl-Gruppe der Formel IIa ist: 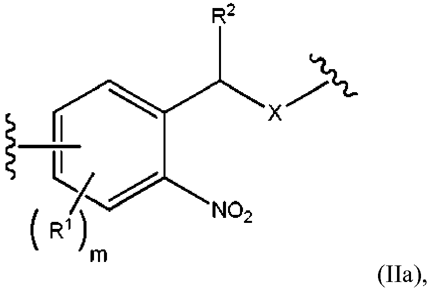 sodass das immobilisierte, photospaltbare Gruppen-Biomolekül-Konjugat eine folgende Struktur aufweist: 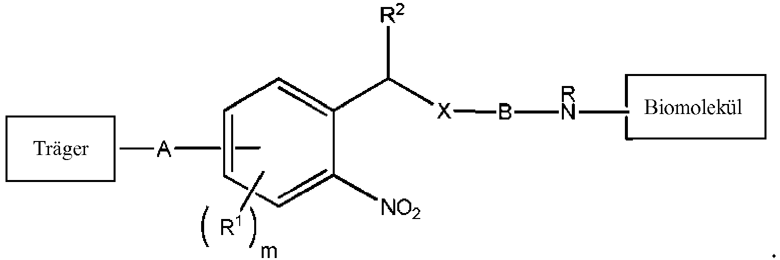 wobei:
jedes R¹ eine Gruppe ist, die unabhängig aus der Gruppe ausgewählt ist, die aus Folgenden besteht: substituiertem oder unsubstituiertem C₁-C₄-Alkyl, substituiertem oder unsubstituiertem C₁-C₄-Halogenalkyl, substituiertem oder unsubstituiertem C₁-C₄-Alkoxyl und substituiertem oder unsubstituiertem C₁-C₄-Halogenalkoxyl;
R² aus der Gruppe ausgewählt ist, die aus Folgenden besteht: H, substituiertem oder unsubstituiertem C₁-C₄-Alkyl und substituiertem oder unsubstituiertem C₁-C₄-Halogenalkyl;
X ist eine Gruppe, die aus der Gruppe ausgewählt ist, die aus Folgenden besteht: -O- und -S-; und
m eine Ganzzahl von 0 bis 3 ist;
wobei jede der vorgenannten Alkyl-, Halogenalkyl-, Alkoxyl- und Halogenalkoxy-Gruppen optional, wo chemisch möglich, durch 1 bis 5 Substituenten substituiert ist, die jeweils unabhängig bei jedem Vorkommen aus der Gruppe ausgewählt sind, die aus Folgenden besteht: Oxo, =NR^{a}, =NOR^{a}, Halo, Nitro, Cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a} CO₂R^{a} C(O)R^{a}, CONR^{a}R^{a}, C₁-C₄-Alkyl, C2-C4-Alkenyl, C₂-C₄-Alkynyl, C₁-C4-Haloalkyl, CR^{b}R^{b}NR^{a}R^{a} und =CR^{b}CR^{b}R^{b}NR^{a}R^{a}; wobei R^{a} bei jedem Vorkommen unabhängig ausgewählt ist aus H, C₁-C₄-Alkyl und C₁-C₄-Haloalkyl; und R^{b} bei jedem Vorkommen unabhängig ausgewählt ist aus H, Halogen, C₁-C₄-Alkyl und C₁-C₄-Haloalkyl; optional
wobei R¹ Methoxy ist; m 1 ist; der R¹-Substituent para für den -NO₂-Substituent ist; R² Methyl ist; und X -O- ist.

9. Verfahren nach einem der Ansprüche 1 bis 7, wobei die photospaltbare Gruppe eine Benzoin-Gruppe der Formel IIb ist: 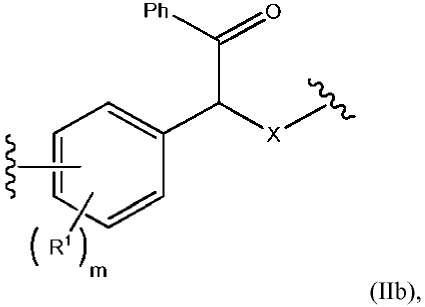 sodass das immobilisierte, photospaltbare Gruppen-Biomolekül-Konjugat eine folgende Struktur aufweist: 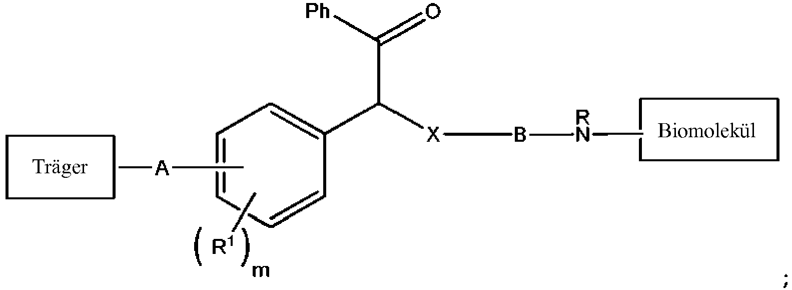 wobei:
jedes R¹ eine Gruppe ist, die unabhängig aus der Gruppe ausgewählt ist, die aus Folgenden besteht: OH, OR' und OCOR'; wobei R¹ aus der Gruppe ausgewählt ist, die aus Folgenden besteht: substituiertem oder unsubstituiertem C₁₋₄-Alkyl, substituiertem oder unsubstituiertem C₁-C₄-Halogenalkyl, substituiertem oder unsubstituiertem Aryl und substituiertem oder unsubstituiertem Heteroaryl;
X eine Gruppe ist, die aus der Gruppe ausgewählt ist, die aus Folgenden besteht: -O- und -S-; und
m eine Ganzzahl von 0 bis 4 ist;
wobei jede der vorgenannten Alkyl-, Halogenalkyl-, Aryl- und Heteroaryl-Gruppen optional, wo chemisch möglich, durch 1 bis 5 Substituenten substituiert ist, die jeweils unabhängig bei jedem Vorkommen aus der Gruppe ausgewählt ist, die aus Folgenden besteht: Oxo, =NR^{a}, =NOR^{a}, Halo, Nitro, Cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a} CO₂R^{a} C(O)R^{a}, CONR^{a}R^{a}, C₁-C₄-Alkyl, C₂-C₄-Alkenyl, C₂-C₄-Alkynyl, C₁-C4-Haloalkyl, CR^{b}R^{b}NR^{a}R^{a} und =CR^{b}CR^{b}R^{b}NR^{a}R^{a}; wobei R^{a} bei jedem Vorkommen unabhängig ausgewählt ist aus H, C₁-C₄-Alkyl und C₁-C₄-Haloalkyl; und R^{b} bei jedem Vorkommen unabhängig ausgewählt aus H, Halogen, C₁-C₄-Alkyl und C₁-C₄-Haloalkyl; optional
wobei R¹ Methoxy ist; m 1 ist; der R¹-Substituent meta für den festen Substituenten der Benzoin-Gruppe der Formel IIb ist; und X -O- ist.

10. Verfahren nach einem der Ansprüche 1 bis 7, wobei die photospaltbare Gruppe eine Phenacylgruppe der Formel IIc ist: 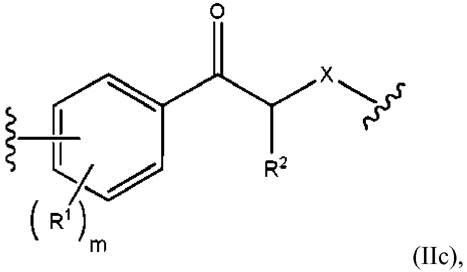 sodass das immobilisierte, photospaltbare Gruppen-Biomolekül-Konjugat eine folgende Struktur aufweist: 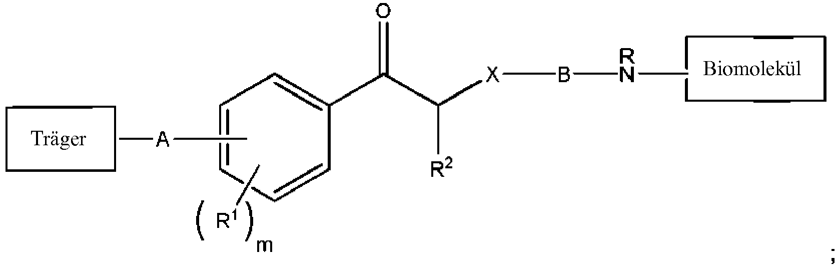 wobei:
jedes R¹ eine Gruppe ist, die unabhängig aus der Gruppe ausgewählt ist, die aus Folgenden besteht: OH, OR' und OCOR'; wobei R¹ aus der Gruppe ausgewählt ist, die aus Folgenden besteht: substituiertem oder unsubstituiertem C₁₋₄-Alkyl, substituiertem oder unsubstituiertem C₁-C₄-Halogenalkyl, substituiertem oder unsubstituiertem Aryl und substituiertem oder unsubstituiertem Heteroaryl;
R² ausgewählt ist von der Gruppe bestehend aus: H, C₁₋₄-Alkyl und C₁-C₄-Halogenalkyl;
X eine Gruppe ist, die aus der Gruppe ausgewählt ist, die aus Folgenden besteht: -O- und -S-; und
m eine Ganzzahl von 0 bis 4 ist;
wobei jede der vorgenannten Alkyl-, Halogenalkyl-, Aryl- und Heteroaryl-Gruppen optional, wo chemisch möglich, durch 1 bis 5 Substituenten substituiert ist, die jeweils unabhängig bei jedem Vorkommen aus der Gruppe ausgewählt sind, die aus Folgenden besteht: Oxo, =NR^{a}, =NOR^{a}, Halo, Nitro, Cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a} CO₂R^{a} C(O)R^{a}, CONR^{a}R^{a}, C₁-C₄-Alkyl, C₂-C₄-Alkenyl, C₂-C₄-Alkynyl, C₁-C₄-Haloalkyl, CR^{b}R^{b}NR^{a}R^{a} und =CR^{b}CR^{b}R^{b}NR^{a}R^{a}; wobei R^{a} bei jedem Vorkommen unabhängig ausgewählt ist aus H, C₁-C₄-Alkyl und C₁-C₄-Haloalkyl; und R^{b} bei jedem Vorkommen unabhängig ausgewählt ist aus H, Halogen, C₁-C₄-Alkyl und C₁-C₄-Haloalkyl; optional
wobei R¹ Methoxy ist; m 1 ist; der R¹-Substituent ortho oder para für den festen Substituenten der Benzoin-Gruppe der Formel IIc ist; R² H ist; und X -O- ist.

11. Verfahren nach einem der Ansprüche 1 bis 7, wobei die photospaltbare Gruppe eine 4-Hydroxyphenacyl-Gruppe der Formel IId ist: 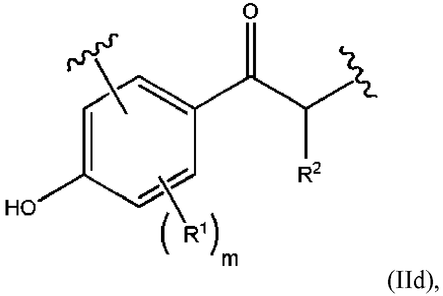 sodass das immobilisierte, photospaltbare Gruppen-Biomolekül-Konjugat eine folgende Struktur aufweist: 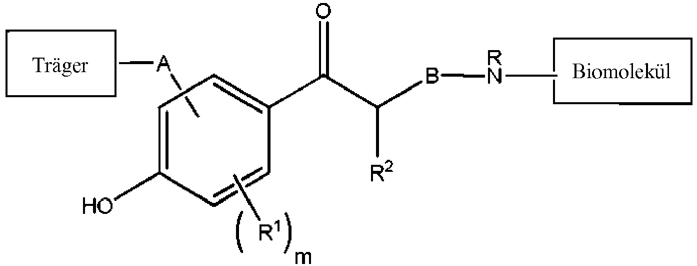 wobei:
jedes R¹ eine Gruppe ist, die unabhängig aus der Gruppe ausgewählt ist, die aus Folgenden besteht: OH, OR' und OCOR'; wobei R' aus der Gruppe ausgewählt ist, die aus Folgenden besteht: substituiertem oder unsubstituiertem C₁₋₄-Alkyl, substituiertem oder unsubstituiertem C₁-C₄-Halogenalkyl, substituiertem oder unsubstituiertem Aryl und substituiertem oder unsubstituiertem Heteroaryl;
R² aus der Gruppe ausgewählt ist, die aus Folgenden besteht: C₁₋₄-Alkyl und C₁-C₄-Haloalkyl; und
m eine Ganzzahl von 0 bis 3 ist;
wobei jede der vorgenannten Alkyl-, Halogenalkyl-, Aryl- und Heteroaryl-Gruppen optional, wo chemisch möglich, durch 1 bis 5 Substituenten substituiert ist, die jeweils unabhängig bei jedem Vorkommen aus der Gruppe ausgewählt sind, die aus Folgenden besteht: Oxo, =NR^{a}, =NOR^{a}, Halo, Nitro, Cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a} CO₂R^{a} C(O)R^{a}, CONR^{a}R^{a}, C₁-C₄-Alkyl, C₂-C₄-Alkenyl, C₂-C₄-Alkynyl, C₁-C₄-Haloalkyl, CR^{b}R^{b}NR^{a}R^{a} und =CR^{b}CR^{b}R^{b}NR^{a}R^{a}; wobei R^{a} bei jedem Vorkommen unabhängig ausgewählt ist aus H, C₁-C₄-Alkyl und C₁-C₄-Haloalkyl; und R^{b} bei jedem Vorkommen unabhängig ausgewählt ist aus H, Halogen, C₁-C₄-Alkyl und C₁-C₄-Haloalkyl; optional
wobei m 0 ist; und R² H ist.

12. Verfahren nach einem der Ansprüche 1 bis 7, wobei die photospaltbare Gruppe eine (2-Hydroxyphenyl)acrylsäure-Gruppe der Formel IIe ist: 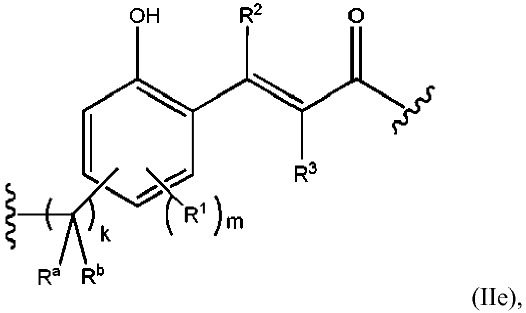 sodass das immobilisierte, photospaltbare Gruppen-Biomolekül-Konjugat eine folgende Struktur aufweist: 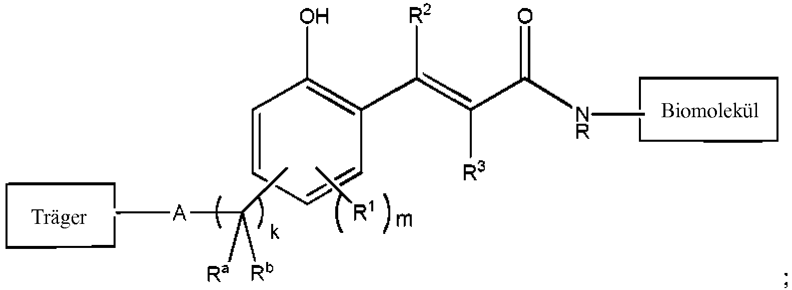 wobei:
jedes R¹ eine Gruppe ist, die unabhängig aus der Gruppe ausgewählt ist, die aus Folgenden besteht: OH, OR' und OCOR'; wobei R' aus der Gruppe ausgewählt ist, die aus Folgenden besteht: substituiertem oder unsubstituiertem C₁₋₄-Alkyl, substituiertem oder unsubstituiertem C₁-C₄-Halogenalkyl, substituiertem oder unsubstituiertem Aryl und substituiertem oder unsubstituiertem Heteroaryl;
R₂ und R₃ jeweils unabhängig aus der Gruppe ausgewählt sind, die aus Folgenden besteht: H, C₁₋₄-Alkyl und C₁-C₄-Haloalkyl;
R^{a} und R^{b} jeweils unabhängig aus der Gruppe ausgewählt sind, die aus Folgenden besteht: H, C₁₋₄-Alkyl und C₁-C₄-Haloalkyl;
k eine Ganzzahl von 0 bis 3 ist; und
m eine Ganzzahl von 0 bis 3 ist;
wobei jede der vorgenannten Alkyl-, Halogenalkyl-, Aryl- und Heteroaryl-Gruppen optional, wo chemisch möglich, durch 1 bis 5 Substituenten substituiert ist, die jeweils unabhängig bei jedem Vorkommen aus der Gruppe ausgewählt ist, die aus Folgenden besteht: Oxo, =NR^{a}, =NOR^{a}, Halo, Nitro, Cyano, NR^{a}R^{a}, NR^{a}S(O)₂R^{a}, NR^{a}CONR^{a}R^{a}, NR^{a}CO₂R^{a}, OR^{a}; SR^{a}, SOR^{a}, SO₃R^{a}, SO₂R^{a}, SO₂NR^{a}R^{a} CO₂R^{a} C(O)R^{a}, CONR^{a}R^{a}, C₁-C₄-Alkyl, C₂-C₄-Alkenyl, C₂-C₄-Alkynyl, C₁-C4-Haloalkyl, CR^{b}R^{b}NR^{a}R^{a} und =CR^{b}CR^{b}R^{b}NR^{a}R^{a}; ; wobei R^{a} bei jedem Vorkommen unabhängig ausgewählt ist aus H, C₁-C₄-Alkyl und C₁-C₄-Haloalkyl; und R^{b} bei jedem Vorkommen unabhängig ausgewählt aus H, Halogen, C₁-C₄-Alkyl und C₁-C₄-Haloalkyl; optional
wobei m 0 ist; k 0 oder 1 ist; R² H ist; R³ H ist; R^{a} H ist; R^{b} H ist.

13. Verfahren nach einem vorherigen Anspruch, wobei B -C(=O)- ist oder nicht vorhanden ist.

14. Verfahren nach einem vorherigen Anspruch, wobei R H ist.

## Revendications

1. Procédé de synthèse d'un conjugué biomolécule-médicament, le procédé comprenant :
(i) soit :
(a) la mise en contact d'une biomolécule ayant un groupe -NRH présent avec un support qui est fonctionnalisé avec un groupe photoclivable dans des conditions adaptées à la liaison par covalence de la biomolécule au groupe photoclivable ; ou
(b) la mise en contact d'une biomolécule ayant un groupe -NRH présent avec un groupe photoclivable dans des conditions adaptées à la liaison par covalence de la biomolécule au groupe photoclivable pour produire un conjugué groupe photoclivable-biomolécule, et puis l'immobilisation du conjugué groupe photoclivable-biomolécule sur un support ;
pour fournir un conjugué groupe photoclivable-biomolécule immobilisé : 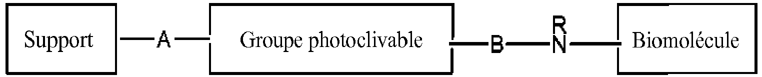
(ii) facultativement la mise en contact du conjugué groupe photoclivable-biomolécule immobilisé avec un agent de modification chimique ou un agent d'activation pour fournir un conjugué groupe photoclivable-biomolécule immobilisé qui est modifié chimiquement ou activé au niveau de la partie biomolécule ;
(iii) la mise en contact du conjugué groupe photoclivable-biomolécule immobilisé de l'étape (i) ou du conjugué groupe photoclivable-biomolécule immobilisé modifié chimiquement ou activé de l'étape (ii) avec un composant de médicament pour former un conjugué groupe photoclivable-bio molécule-médicament immobilisé : 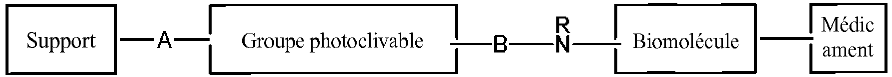
(iv) l'irradiation du conjugué groupe photoclivable-biomolécule-médicament immobilisé de l'étape (iii) avec un rayonnement libérant ainsi le conjugué biomolécule-médicament 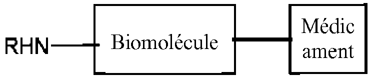 dans lequel :
-A- est absent ou est un groupe espaceur ;
-B- est absent ou est un groupe espaceur ;
un 'groupe photoclivable' comprend tout résidu qui est capable de se cliver à l'exposition à un rayonnement pour permettre la libération du conjugué biomolécule-médicament ;
R est sélectionné parmi le groupe constitué par : H, un alkyle en C₁ à C₈, un alcényle en C₂ à C₈, un alcynyle en C₂ à C₈, un haloalkyle en C₁ en C₈, -(CR^{a}R^{a})ₙ-cycloalkyle en C₃ à C₆, -(CR^{a}R^{a})ₙ-hétérocycloalkyle en C₃ à C₆, -(CR^{a}R^{a})ₙ-halocycloalkyle en C₃ à C₆, -(CR^{a}R^{a})ₙ-aryle, et -(CR^{a}R^{a})ₙ-hétéroaryle ;
R^{a} est indépendamment à chaque occurrence sélectionné parmi : H, Me, CF₃ et F ;
n est un nombre entier sélectionné indépendamment à chaque occurrence parmi 0, 1, 2 et 3 ; et
un 'médicament' est un résidu d'une molécule de médicament sélectionné parmi le groupe constitué par des inhibiteurs de la tubuline et des agents d'interaction avec l'ADN.

2. Procédé selon la revendication 1, dans lequel l'étape (i) comprend l'incubation pendant une période de temps d'environ 10 minutes à environ 1 jour à une température d'environ 10 °C à environ 30 °C.

3. Procédé selon l'une quelconque des revendications précédentes, dans lequel l'étape (ii) est obligatoire et comprend la réduction de la biomolécule ; facultativement
dans lequel l'étape (ii) comprend la réduction de la biomolécule avec de la tris(2-carboxyethyl)phosphine (TCEP).

4. Procédé selon l'une quelconque des revendications précédentes, dans lequel l'étape (iii) comprend la mise en contact avec un composant médicament dans une solution tampon.

5. Procédé selon l'une quelconque des revendications précédentes, dans lequel l'étape (iv) comprend l'irradiation avec une lumière UV ayant une longueur d'onde d'environ 320 nm à environ 400 nm, préférablement une longueur d'onde d'environ 365 nm.

6. Procédé selon l'une quelconque des revendications précédentes, dans lequel le support est une bille ; et/ou est à base d'agarose.

7. Procédé selon l'une quelconque des revendications précédentes, dans lequel la biomolécule est sélectionnée parmi le groupe comprenant : des anticorps, des fragments d'anticorps, des anticorps modifiés, des enzymes, des protéines, des peptides, des polypeptides, des peptides modifiés, des acides nucléiques peptidiques (ANP), des métalloprotéines, des hybrides peptide-oligonucléotide, des acides aminés, des acides aminés d'origine non naturelle ; des acides diaminés, des acides aminés synthétiques, des oligonucléotides, des oligonucléotide modifiés, des nucléotides, des nucléosides, des purines, des pyrimidines, des oligosaccharides, un polysaccharide, des disaccharides, des monosaccharides, des sucres aminés, des lipides, des phospholipides, des glycolipides, des stérols, des vitamines, des hormones, des stéroïdes, des neurotransmetteurs, des carbohydrates, des sucres, des virus, des cellules, des ingrédients pharmaceutiques actifs (IPA) et des composés précurseurs ou un dérivé de l'un quelconque de ceux-ci.

8. Procédé selon l'une quelconque des revendications 1 à 7, dans lequel le groupe photoclivable est un groupe 2-nitrophenzyle de Formule IIa : 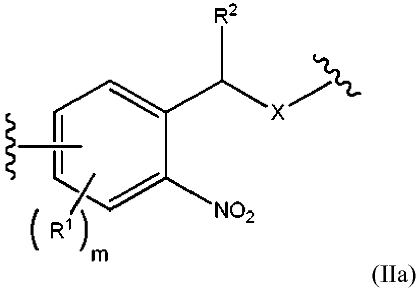 de sorte que le conjugué groupe photoclivable-biomolécule immobilisé a la structure : 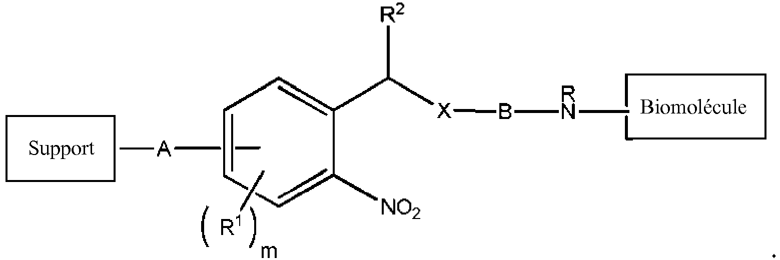 dans lequel :
chaque R¹ est un groupe sélectionné indépendamment parmi le groupe constitué par : un alkyle en C₁ à C₄ substitué ou non substitué, un haloalkyle en C₁ à C₄ substitué ou non substitué, un alcoxyle en C₁ à C₄ substitué ou non substitué et un haloalcoxyle en C₁ à C₄ substitué ou non substitué ;
R² est sélectionné parmi le groupe constitué par : H, un alkyle en C₁ à C₄ substitué ou non substitué et un haloalkyle en C₁ à C₄ substitué ou non substitué ;
X est un groupe sélectionné parmi le groupe constitué par -O- et -S- ; et m est un nombre entier de 0 à 3 ;
dans lequel chacun des groupes alkyle, haloalkyle, alcoxyle et haloalcoxyle susmentionnés est facultativement substitué, lorsque c'est possible chimiquement, par 1 à 5 substituants qui sont chacun indépendamment à chaque occurrence sélectionnés parmi le groupe constitué par : oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}, SR^{a}, SOR^{a}, SO₃R^{a}, SO₂R^{a}, SO₂NR^{a}R^{a}, CO₂R^{a} C(O)R^{a}, CONR^{a}R^{a}, un alkyle en C₁ à C₄, un alcényle en C₂ à C₄, un alcynyle en C₂ à C₄, un haloalkyle en C₁ à C₄, CR^{b}R^{b}NR^{a}R^{a}, et =CR^{b}CR^{b}R^{b}NR^{a}R^{a} ; dans lequel R^{a} est indépendamment à chaque occurrence sélectionné parmi H, un alkyle en C₁ à C₄ et un haloalkyle en C₁ à C₄ ; et R^{b} est indépendamment à chaque occurrence sélectionné parmi H, un halogène, un alkyle en C₁ à C₄ et un haloalkyle en C₁ à C₄ ; facultativement
dans lequel R¹ est un méthoxy ; m est 1 ; le substituant R¹ est para par rapport au substituant -NO₂ ; R² est un méthyle ; et X est -O-.

9. Procédé selon l'une quelconque des revendications 1 à 7, dans lequel le groupe photoclivable est un groupe benzoïne de Formule IIb : 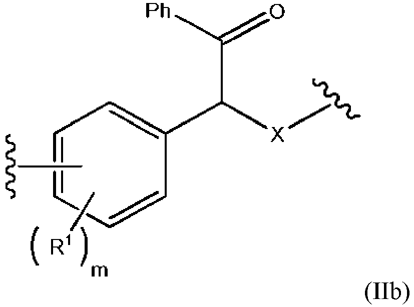 de sorte que le conjugué groupe photoclivable-biomolécule immobilisé a une structure : 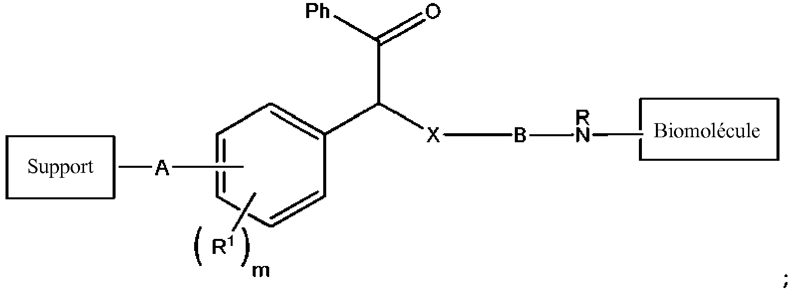 dans lequel :
chaque R¹ est un groupe sélectionné indépendamment parmi le groupe constitué par : OH, OR' et OCOR' ; dans lequel R' est sélectionné parmi le groupe constitué par : un alkyle en C₁ à C₄ substitué ou non substitué, un haloalkyle en C₁ à C₄ substitué ou non substitué, un aryle substitué ou non substitué et un hétéroaryle substitué ou non substitué ;
X est un groupe sélectionné parmi le groupe constitué par -O- et -S- ; et m est un nombre entier de 0 à 4 ;
dans lequel chacun des groupes alkyle, haloalkyle, aryle et hétéroaryle susmentionnés est facultativement substitué, lorsque c'est possible chimiquement, par 1 à 5 substituants qui sont chacun indépendamment à chaque occurrence sélectionnés parmi le groupe constitué par : oxo, =NR^{a}, =NOR^{a}, un halo, un nitro, un cyano, NR^{a}R^{a}, NR^{a}S(O)₂R^{a}, NR^{a}CONR^{a}R^{a}, NR^{a}CO₂R^{a}, OR^{a}, SR^{a}, SOR^{a}, SO₃R^{a}, SO₂R^{a}, SO₂NR^{a}R^{a}, CO₂R^{a} C(O)R^{a}, CONR^{a}R^{a}, un alkyle en C₁ à C₄, un alcényle en C₂ à C₄, un alcynyle en C₂ à C₄, un haloalkyle en C₁ à C₄, CR^{b}R^{b}NR^{a}R^{a}, et =CR^{b}CR^{b}R^{b}NR^{a}R^{a} ; dans lequel R^{a} est indépendamment à chaque occurrence sélectionné parmi H, un alkyle en C₁ à C₄ et un haloalkyle en C₁ à C₄ ; et R^{b} est indépendamment à chaque occurrence sélectionné parmi H, un halogène, un alkyle en C₁ à C₄ et un haloalkyle en C₁ à C₄; facultativement
dans lequel R¹ est un méthoxy ; m est 1 ; le substituant R¹ est méta par rapport au substituant fixe du groupe benzoïne de Formule IIb ; et X est -O-.

10. Procédé selon l'une quelconque des revendications 1 à 7, dans lequel le groupe photoclivable est un groupe phénacyle de Formule IIc : 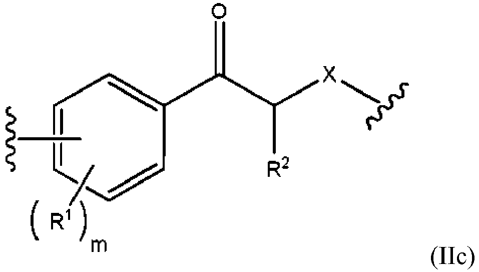 de sorte que le conjugué groupe photoclivable-biomolécule immobilisé a une structure : 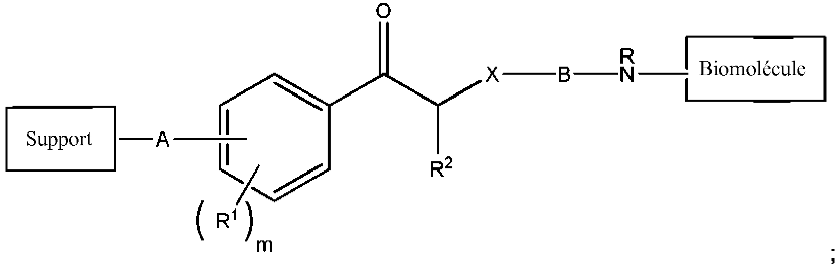 dans lequel :
chaque R¹ est un groupe sélectionné indépendamment parmi le groupe constitué par : OH, OR' et OCOR' ; dans lequel R' est sélectionné parmi le groupe constitué par : un alkyle en C₁ à C₄ substitué ou non substitué, un haloalkyle en C₁ à C₄ substitué ou non substitué, un aryle substitué ou non substitué et un hétéroaryle substitué ou non substitué ;
R² est sélectionné parmi le groupe constitué par : H, un alkyle en C₁ à C₄ et un haloalkyle en C₁ à C₄ ;
X est un groupe sélectionné parmi le groupe constitué par : -O- et -S- ; et
m est un nombre entier de 0 à 4 ;
dans lequel chacun des groupes alkyle, haloalkyle, aryle et hétéroaryle susmentionnés est facultativement substitué, lorsque c'est possible chimiquement, par 1 à 5 substituants qui sont chacun indépendamment à chaque occurrence sélectionnés parmi le groupe constitué par: oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a} ; SR^{a}, SOR^{a}, SO₃R^{a}, SO₂R^{a}, SO₂NR^{a}R^{a}, CO₂R^{a} C(O)R^{a}, CONR^{a}R^{a}, un alkyle en C₁ à C₄, un alcényle en C₂ à C₄, un alcynyle en C₂ à C₄, un haloalkyle en C₁ à C₄, CR^{b}R^{b}NR^{a}R^{a}, et =CR^{b}CR^{b}R^{b}NR^{a}R^{a} ; dans lequel R^{a} est indépendamment à chaque occurrence sélectionné parmi H, un alkyle en C₁-C₄ et un haloalkyle en C₁ à C₄ ; et R^{b} est indépendamment à chaque occurrence sélectionné parmi H, un halogène, un alkyle en C₁ à C₄ et un haloalkyle en C₁ à C₄ ; facultativement
dans lequel R¹ est un méthoxy ; m est 1 ; le substituant R¹ est ortho or para par rapport au substituant fixe du groupe benzoïne de Formule IIc ; R² est H ; et X est -O-.

11. Procédé selon l'une quelconque des revendications 1 à 7, dans lequel le groupe photoclivable est un groupe 4-hydroxy phénacyle de Formule IId : 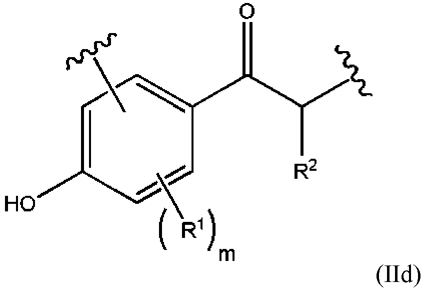 de sorte que le conjugué groupe photoclivable-biomolécule immobilisé a une structure : 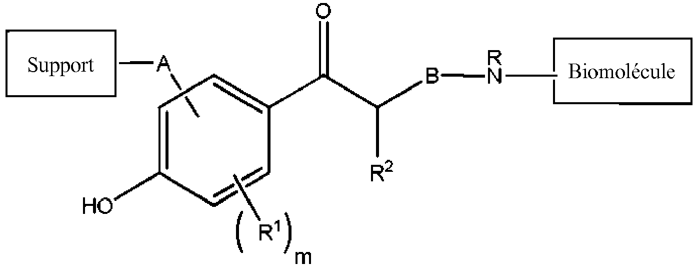 dans lequel :
chaque R¹ est un groupe sélectionné indépendamment parmi le groupe constitué par : OH, OR' et OCOR' ; dans lequel R' est sélectionné parmi le groupe constitué par : un alkyle en C₁ à C₄ substitué ou non substitué, un haloalkyle en C₁ à C₄ substitué ou non substitué, un aryle substitué ou non substitué et un hétéroaryle substitué ou non substitué ;
R² est sélectionné parmi le groupe constitué par : un alkyle en C₁ à C₄ et un haloalkyle en C₁ à C₄ ; et
m est un nombre entier de 0 à 3 ;
dans lequel chacun des groupes alkyle, haloalkyle, aryle et hétéroaryle susmentionnés est facultativement substitué, lorsque c'est possible chimiquement, par 1 à 5 substituants qui sont chacun indépendamment à chaque occurrence sélectionnés parmi le groupe constitué par : oxo, =NR^{a}, =NOR^{a}, halo, nitro, cyano, NR^{a}R^{a}, NR^{a}S(O)₂R^{a}, NR^{a}CONR^{a}R^{a}, NR^{a}CO₂R^{a}, OR^{a}, SR^{a}, SOR^{a}, SO₃R^{a}, SO₂R^{a}, SO₂NR^{a}R^{a}, CO₂R^{a} C(O)R^{a}, CONR^{a}R^{a}, un alkyle en C₁ à C₄, un alcényle en C₂ à C₄, un alcynyle en C₂ à C₄, un haloalkyle en C₁ à C₄, CR^{b}R^{b}NR^{a}R^{a}, et =CR^{b}CR^{b}R^{b}NR^{a}R^{a} ; dans lequel R^{a} est indépendamment à chaque occurrence sélectionné parmi H, un alkyle en C₁ à C₄ et un haloalkyle en C1 à C₄ ; et R^{b} est indépendamment à chaque occurrence sélectionné parmi H, un halogène, un alkyle en C₁ à C₄ et un haloalkyle en C₁ à C₄ ; facultativement
dans lequel m est 0 ; et R² est H.

12. Procédé selon l'une quelconque des revendications 1 à 7, dans lequel le groupe photoclivable est un groupe acide (2-hydroxyphényl)acrylique de Formule Ile : 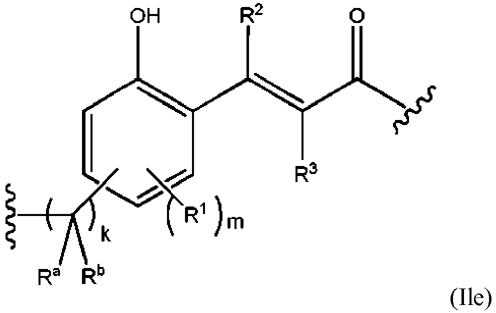 de sorte que le conjugué groupe photoclivable-biomolécule immobilisé a une structure : 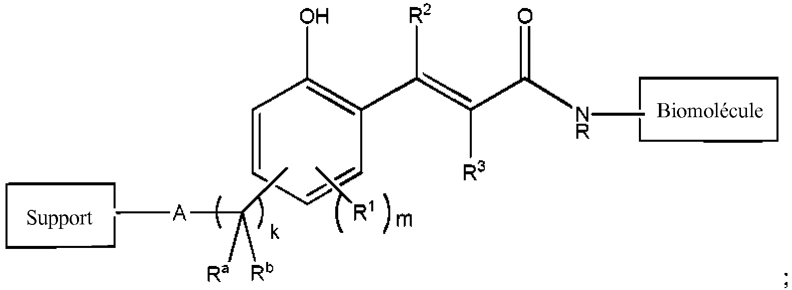 dans lequel :
chaque R¹ est un groupe sélectionné indépendamment parmi le groupe constitué par : OH, OR' et OCOR' ; dans lequel R' est sélectionné parmi le groupe constitué par : un alkyle en C₁ à C₄ substitué ou non substitué, un haloalkyle en C₁ à C₄ substitué ou non substitué, un aryle substitué ou non substitué et un hétéroaryle substitué ou non substitué ;
R² et R³ sont chacun indépendamment sélectionnés parmi le groupe constitué par : H, un alkyle en C₁ à C₄ et un haloalkyle en C₁ à C₄ ;
R^{a} et R^{b} sont chacun indépendamment sélectionnés parmi le groupe constitué par : H, un alkyle en C₁ à C₄ et un haloalkyle en C₁ à C₄ ;
k est un nombre entier de 0 à 3 ; et
m est un nombre entier de 0 à 3 ;
dans lequel chacun des groupes alkyle, haloalkyle, aryle et hétéroaryle susmentionnés est facultativement substitué, lorsque c'est possible chimiquement, par 1 à 5 substituants qui sont chacun indépendamment à chaque occurrence sélectionnés parmi le groupe constitué par : oxo, =NR^{a}, =NOR^{a}, un halo, un nitro, un cyano, NR^{a}R^{a}, NR^{a}S(O)₂R^{a}, NR^{a}CONR^{a}R^{a}, NR^{a}CO₂R^{a}, OR^{a}, SR^{a}, SOR^{a}, SO₃R^{a}, SO₂R^{a}, SO₂NR^{a}R^{a}, CO₂R^{a} C(O)R^{a}, CONR^{a}R^{a}, un alkyle en C₁ à C₄, un alcényle en C₂ à C₄, un alcynyle en C₂ à C₄, un haloalkyle en C₁ à C₄, CR^{b}R^{b}NR^{a}R^{a}, et =CR^{b}CR^{b}R^{b}NR^{a}R^{a} ; dans lequel R^{a} est indépendamment à chaque occurrence sélectionné parmi H, un alkyle en C₁ à C₄ et un haloalkyle en C₁ à C₄; et R^{b} est indépendamment à chaque occurrence sélectionné parmi H, un halogène, un alkyle en C₁ à C₄ et un haloalkyle en C₁ à C₄; facultativement
dans lequel m est 0 ; k est 0 ou 1 ; R² est H ; R³ est H ; R^{a} est H ; R^{b} est H.

13. Procédé selon l'une quelconque des revendications, dans lequel B est -C(=O)- ou absent.

14. Procédé selon l'une quelconque des revendications, dans lequel R est H.
